(19) Europäisches Patentamt
European Patent Office
Office européen des brevets

(11) EP 3 238 600 A1

(12) EUROPEAN PATENT APPLICATION
published in accordance with Art. 153(4) EPC

(43) Date of publication:
01.11.2017 Bulletin 2017/44

(21) Application number: 15872962.4

(22) Date of filing: 21.12.2015

(51) Int Cl.:
*A47L 25/00* (2006.01) *C09J 11/06* (2006.01)
*C09J 133/00* (2006.01)

(86) International application number:
PCT/JP2015/085616

(87) International publication number:
WO 2016/104394 (30.06.2016 Gazette 2016/26)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 22.12.2014 JP 2014259505

(71) Applicants:
- **Kabushiki Kaisha Nitoms**
  **Tokyo 140-0002 (JP)**
- **Nitto Denko Corporation**
  **Ibaraki-shi, Osaka 567-8680 (JP)**

(72) Inventors:
- **SUYAMA, Yousuke**
  **Shinagawa-ku, Tokyo 1400002 (JP)**
- **SAKASHITA, Teiji**
  **Shinagawa-ku, Tokyo 1400002 (JP)**
- **KAWAI, Yumi**
  **Shinagawa-ku, Tokyo 1400002 (JP)**
- **KOJIMA, Takenobu**
  **Shinagawa-ku, Tokyo 1400002 (JP)**
- **TERADA, Itsumi**
  **Shinagawa-ku, Tokyo 1400002 (JP)**

(74) Representative: **Müller-Boré & Partner Patentanwälte PartG mbB**
**Friedenheimer Brücke 21**
**80639 München (DE)**

# (54) ADHESIVE CLEANER FOR USE IN ANTIBACTERIAL TREATMENT

(57) Provided is a sticky cleaner for antimicrobial treatment capable of removing organic dirt deposited on an article surface and of providing antimicrobial properties to the surface. The sticky cleaner according to this invention comprises a dirt-collecting member that collects organic dirt as it comes in contact with the article surface. The dirt-collecting member comprises a PSA in an area that comes in contact with the article surface, with the PSA providing antimicrobial properties to the article surface upon contact with the surface. The PSA comprises a base polymer and an antimicrobial agent.

FIG.1

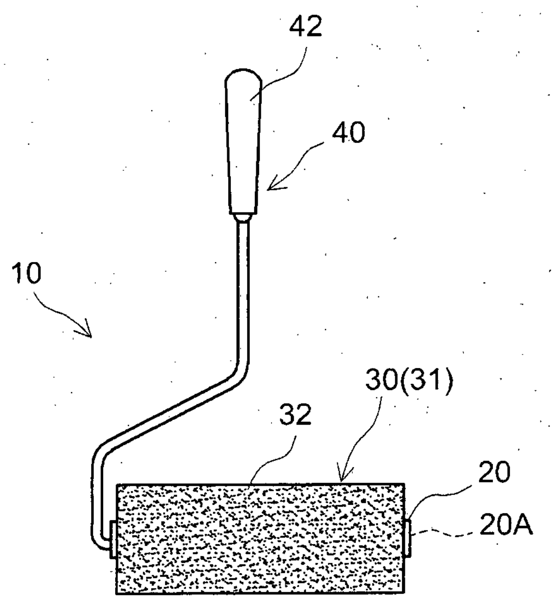


EP 3 238 600 A1

## Description

[Technical Field]

The present invention relates to a sticky cleaner for antimicrobial treatment used for removal of organic dirt on an article surface and for antimicrobial treatment of the surface.

The present application claims priority based on Japanese Patent Application No. 2014-259505 filed on December 22, 2014 and the entire contents thereof are incorporated herein by reference.

[Background Art]

Displays typically formed of liquid crystal panels or an organic EL (OEL) panels are placed on surfaces of portable devices, for instance, portable PCs such as notebook PCs; tablet terminals such as electronic books; mobile phones such as smartphones; mobile gaming devices; various types of PDA (personal digital assistant); and the like. These portable devices are carried and used on a daily basis, and thus easily attract dust and organic dirt such as finger marks, cosmetics and sebum. In particular, recently wide-spread touch-screen portable devices include display/input members and are operated with a direct finger touch by a user to the display/input members with the displays functioning also as input devices, and thus are more likely to attract organic dirt such as finger marks, sebum, etc. Not just these portable devices, but also show window glass, glass tables, showcases and the like accumulate organic dirt, leading to degradation of their exterior to make them unsightly. To tackle such organic dirt, sticky cleaners are suggested, which remove organic dirt from an article surface upon contact therewith (Patent Documents 1 and 2).

[Citation List]

[Patent Literature]

[Patent Document 1] WO 2013/015075
[Patent Document 2] WO 2014/115632

[Summary of Invention]

[Technical Problem]

In general, the articles that easily attract organic dirt often happen to come in contact with people's fingers and faces as well. In association with increased awareness of hygiene in late years, these articles tend to be expected to give feelings of cleanliness even in aspects other than visible dirt. Thus, it is beneficial to provide a sticky cleaner capable of not only removing organic dirt from an article surface, but also providing antimicrobial properties to the surface.

The present invention has been made in view of such circumstances with an objective to provide a sticky cleaner for antimicrobial treatment, which can remove organic dirt on an article surface and also provide antimicrobial properties to the surface.

[Solution to Problem]

The present invention provides a sticky cleaner for antimicrobial treatment used for removal of organic dirt deposited on an article surface and for antimicrobial treatment of the surface. The sticky cleaner comprises a dirt-collecting member that collects organic dirt as it comes in contact with the article surface. The dirt-collecting member comprises a pressure-sensitive adhesive (PSA) in an area that comes in contact with the article surface, with the PSA providing antimicrobial properties to the article surface upon contact therewith. The PSA comprises a base polymer and an antimicrobial agent.

The sticky cleaner is in an embodiment such that by using the dirt-collecting member that catches organic dirt on an article surface (a surface subject to cleaning), in particular, by allowing the PSA of the dirt-collecting member to make contact with the surface subject to cleaning, antimicrobial properties can be provided to the surface being cleaned. The sticky cleaner in such an embodiment can be applied to the surface subject to cleaning, thereby to remove organic dirt thereon and to provide antimicrobial treatment thereto.

The surface-contacting part of the dirt-collecting member preferably exhibits an adhesive strength of 1 N/25 mm or less. This means that the dirt-collecting member is easy-release. A cleaner having such an easy-release dirt-collecting member provides excellent dirt-removing workability since it requires little force to do the dirt-removing work on the surface. Because of the easy-release nature, even when the article surface (e.g. tablet terminal's display) is covered with removable protection film, the protection film is less likely to get peeled off while cleaning the article surface covered with the protection film (i.e. the surface of the protection film). Despite of the easy-release nature as described above, the sticky cleaner for antimicrobial treatment disclosed herein can effectively remove organic dirt from an article surface and easily provide antibacterial properties to the surface.

As described above, the organic dirt includes sebum from the skin; and as evident from this, it may include inorganic substances such as sodium and potassium as well as their salts. The adhesive strength refers to the 180° peel strength to SUS determined based on the 180° peel test described later.

In a preferable embodiment of the art disclosed herein, the PSA comprises 2 % to 50 % (by weight) antimicrobial agent. The sticky cleaner for antimicrobial treatment having a PSA with such a composition more favorably combines an ability to remove organic dirt deposited on an article surface and an ability to provide antimicrobial properties to the surface.

The PSA preferably comprises a plasticizer in addition to the base polymer and the antimicrobial agent. With the inclusion of the plasticizer in the PSA, organic dirt on the surface being cleaned can be more favorably collected. The inclusion of the plasticizer may also facilitate the release (separation) from the surface being cleaned to increase the dirt-collecting workability. The PSA includes the antimicrobial agent so that, in the composition comprising the plasticizer, it is able to provide significant antimicrobial properties to the surface being cleaned. This can bring about a favorable combination of an ability to remove organic dirt deposited on an article surface and an ability to provide antimicrobial properties to the surface.

In a preferable embodiment of the art disclosed herein, the PSA may comprise 5 parts to 100 parts by weight of the plasticizer to 100 parts by weight of the base polymer. The sticky cleaner for antimicrobial treatment having a PSA with such a composition can combine more favorably an ability to remove organic dirt deposited on an article surface and an ability to provide antimicrobial properties to the surface.

When the PSA comprises a plasticizer, the antimicrobial agent content Wa to the plasticizer content Wp ratio (Wa/Wp) by weight in the PSA is preferably 0.10 to 1.0. The sticky cleaner for antimicrobial treatment that comprises a PSA comprising a plasticizer and an antimicrobial agent at such a ratio can include the plasticizer in an amount suited for facilitating the release of the dirt-collecting member while favorably providing antimicrobial properties to the surface being cleaned.

The PSA preferably comprises an acrylic polymer as the base polymer. The use of the PSA having such a composition can favorably bring about a sticky cleaner for antimicrobial treatment that favorably achieves both organic dirt removal and easy release while being suited for providing antimicrobial properties.

A preferable example of the base polymer is an acrylic block copolymer having a hard segment (A) and a soft segment (B) in one molecule. With a PSA having such a composition, it is possible to make a sticky cleaner for antimicrobial treatment that favorably combines organic dirt removability and easy-release nature while being suited for providing antimicrobial properties. The PSA with such a composition is likely to be easy to use (easy to apply, etc.) as a hot melt PSA composition. This is preferable from the standpoint of the productivity of the sticky cleaner for antimicrobial treatment, the handling properties of the PSA composition, etc.

The monomer units forming the soft segment (B) preferably comprise an alkyl acrylate having an alkyl group with 6 or more carbon atoms. With a PSA that comprises such an acrylic block copolymer as the base polymer, a sticky cleaner for antimicrobial treatment having greater properties (e.g. excellent organic dirt removability) can be made. A preferable example of the alkyl acrylate having an alkyl group with 6 or more carbon atoms is 2-ethylhexyl acrylate (2EHA).

In a preferable embodiment of the sticky cleaner for antimicrobial treatment disclosed herein, the sticky cleaner comprises a cylindrical rolling member, and the dirt-collecting member is arranged along the lateral surface of the rolling member. According to the sticky cleaner for antimicrobial treatment in such an embodiment, when the rolling member is allowed to rotate around the axis of the cylinder, the dirt-collecting member placed on the lateral surface can efficiently collect organic dirt deposited on an article surface to remove it from the surface while efficiently allowing the PSA to make contact with the surface to provide antimicrobial properties thereto.

The sticky cleaner for antimicrobial treatment disclosed herein may further comprise a grip member that supports the rolling member in a freely rotatable manner. With this configuration, a user can hold the grip member and rotate the rolling member to efficiently remove organic dirt from the article surface while efficiently providing antimicrobial treatment to the surface.

A sticky cleaner for antimicrobial treatment having the prescribed configuration disclosed herein can effectively remove organic dirt (typically sebum dirt of animal origin, e.g. human sebum dirt). Accordingly, a preferable embodiment of the sticky cleaner for antimicrobial treatment disclosed herein is a sticky cleaner used for removing sebum dirt as the organic dirt. The sebum dirt in this description refers to dirt comprising sebum and means to encompass dirt being a mixture of sebum and other organic or inorganic components. Thus, the concept of sebum dirt may include dirt from hands and fingerprints as well as, for instance, a mixture of sebum and inorganic components such as pigment in

foundation cream, a mixture of sebum and inorganic components in perspiration such as sodium chloride, a mixture of sebum and organic components such as of moisturizing cream and sunscreen, etc.

In a preferable embodiment of the sticky cleaner for antimicrobial treatment disclosed herein, the article is a portable device having a display with a surface (typically a smooth, flat surface) formed of glass or synthetic resin. The portable device is carried and used on a daily basis and is likely to attract organic dirt such as finger marks, cosmetics and sebum. In particular, a portable device having a touch panel display (display/input member) is operated with a direct finger touch to the display/input portion, and thus is more likely to attract organic dirt such as finger marks, cosmetics, sebum, etc. The sticky cleaner disclosed herein can easily remove such organic dirt and also easily provide antimicrobial properties to the surface to be cleaned; and therefore, it is particularly preferably used for dirt removal and antimicrobial treatment of a portable device having a display as described above (e.g. a touch panel display).

[Brief Description of Drawings]

Fig. 1 shows a front view schematically illustrating the sticky cleaner for antimicrobial treatment according to an embodiment.

Fig. 2 shows a side view schematically illustrating the sticky cleaner for antimicrobial treatment according to an embodiment.

Fig. 3 shows a cross-sectional view schematically illustrating the dirt-collecting member constituting the sticky cleaner for antimicrobial treatment according to an embodiment.

Fig. 4 shows a perspective view schematically illustrating an example of usage of the sticky cleaner for antimicrobial treatment according to an embodiment.

Fig. 5 shows a diagram schematically illustrating the mechanism of dirt-removing ability recovery effect of the sticky cleaner for antimicrobial treatment according to an embodiment.

[Description of Embodiments]

Preferred embodiments of the present invention are described below. The present invention is not, however, limited to these embodiments. Matters necessary to practice this invention other than those specifically referred to in this description may be understood as design matters to a person of ordinary skill in the art based on the conventional art in the pertinent field. The present invention can be practiced based on the contents disclosed in this description and common technical knowledge in the subject field. In the drawings referenced below, a common reference numeral may be assigned to members or sites producing the same effects, and duplicated descriptions may be omitted or simplified.

The article on which the sticky cleaner for antimicrobial treatment disclosed herein is used is not particularly limited. An article having a smooth surface (typically a smooth, flat surface) is preferable. Examples of such an article include show window glass, glass tables, showcases, mirrors, water tanks (aquariums), various types of display (displays of mounted, free-standing or portable TVs and PCs, etc.), and various types of devices having touch panel display/input members (e.g. free-standing or portable devices such as automated teller machines (ATM), operation terminals of vehicle navigation systems, guide boards, etc.). As organic dirt stuck on their smooth surfaces (e.g. transparent glass surfaces) is unsightly, if found any, its quick removal is desirable. Because these surfaces may be touched by fingers or brought near a face, providing antimicrobial properties can give feelings of cleanliness and safety. Thus, on their surfaces, the sticky cleaner for antimicrobial treatment disclosed herein can be preferably used.

Preferable examples of the article on which the sticky cleaner for antimicrobial treatment disclosed herein is used include various portable devices. The term portable device herein refers to a mobile device and is not limited to a particular device. A preferable portable device has a smooth surface (typically a smooth, flat surface) at least partially on the outside. Examples of such a portable device include portable devices such as portable PCs such as notebook PCs; tablet terminals such as electronic books, etc.; smartphones and other mobile phones; mobile gaming devices; PDAs (personal digital assistants) such as electronic organizers; digital cameras, digital photo frames, hand-held mirrors and the like. Since these are carried and used on a daily basis, they are likely to attract dust and especially organic dirt such as finger marks, cosmetics and sebum. Some of these portable devices have smooth surfaces (typically glass or synthetic resin surfaces) serving as displays such as liquid crystal displays and OEL displays. Organic dirt on the displays makes information displayed thereon hard to see, thereby hindering the use. Moreover, depending on the amount of organic dirt stuck thereon, it might give a filthy impression. On portable devices having such displays, the sticky cleaner for antimicrobial treatment disclosed herein can be preferably used.

Devices having touch panel display/input portions are likely to attract the organic dirt since users directly touch the displays with fingers. Like so, they are directly touched by fingers; and therefore, providing antimicrobial properties can provide users with feelings of cleanliness and safety. Thus, the sticky cleaner for antimicrobial treatment disclosed

herein can be preferably used on them. Among them, tablet terminals such as electronic books and the like have relatively large displays; and therefore, they are considered as particularly preferable articles on which the sticky cleaner for antimicrobial treatment is used.

The sticky cleaner for antimicrobial treatment disclosed herein can be preferably used on a smartphone and other mobile phone. When a mobile phone is brought close to one's ear and mouth for a telephone call, etc., it is likely to accumulate organic dirt such as facial sebum and cosmetics on contact with the face. Since the device is used in contact with or in the vicinity of a face, it is greatly significant to remove organic dirt from the surface and provide antimicrobial properties, thereby to provide users with feelings of cleanliness and safety. Thus, these devices are thought as particularly preferable objects on which the sticky cleaner for antimicrobial treatment is used.

The sticky cleaner for antimicrobial treatment according to an embodiment is described below with reference to drawings. As shown in Figs. 1 and 2, a sticky cleaner for antimicrobial treatment (or simply cleaner, hereinafter) 10 comprises a cylindrical holding member (core) 20 and a PSA sheet roll 30 held on the outer periphery of holding member 20. These holding member 20 and PSA sheet roll 30 are assembled to form a cylindrical rolling member. The holding member material is not particularly limited. A holding member made of a polyolefin or other synthetic resin as well as paper can be preferably used.

Cleaner 10 further comprises a grip member 40 in a form of a pole that supports holding member 20 in a freely rotatable manner. In particular, holding member 20 has a center hole (not shown in the drawings) formed where the central axis of the cylinder is located. A terminal section (one end) of grip member 40 is inserted through the center hole, whereby holding member 20 is installed on grip member 40 in a freely rotatable manner. To the other end of grip member 40, a handle 42 is attached. The materials of grip member and handle are not particularly limited. For instance, metal or synthetic resin pieces can be used.

PSA sheet roll 30 in cleaner 10 is formed by winding a PSA sheet 31 that serves as the dirt-collecting member. In particular, PSA sheet (dirt-collecting member) 31 is configured as a single-faced PSA sheet 31 comprising, as shown in Fig. 3, a support substrate 36 in a form of a long sheet (band) and a PSA layer 32 placed on one face 36A of the support substrate 36. Single-faced PSA sheet 31 is wound with the PSA layer 32 on the outside and formed as a PSA sheet roll 30.

With respect to cleaner 10 having a constitution as described above, some applications are now described. As shown in Fig. 4, cleaner 10 is used for removing organic dirt stuck on a display 2 of a portable device 1 and for providing antimicrobial treatment to the surface of display 2. Display 2 in portable device 1 has a smooth, flat surface. An operator places cleaner 10 on the display 2 of portable device 1, grip a handle 42, and applies a prescribed amount of external force to cleaner 10. The force is then transmitted from grip member 40 to holding member 20, and the PSA layer 32 (a section of the dirt-collecting member that makes contact with the smooth surface) placed on the outer periphery of holding member 20 rotationally moves on display 2. In Fig. 4, PSA sheet roll 30 moves on display 2 in the direction shown by the arrow. During this, the PSA layer 32 collects dust, fine particles, and especially organic dirt (e.g. sebum dirt such as sebum-containing dirt from hands, fingerprints, etc.) present on the display 2. By this means, cleaning (dirt removal) of display 2 is easily and certainly accomplished in the rotation direction of PSA sheet roll 30 (more specifically, the PSA sheet (dirt-collecting member) 31). In dirt removal involving a contact between the PSA layer 32 and the display 2 as described above, at least some of the antimicrobial component(s) (typically an organic antimicrobial agent) in the PSA layer 32 is transferred from the surface of the PSA layer 32 to the surface of the display 2 to remain on the surface of the display 2 even after the cleaner 10 leaves the display 2. With the antimicrobial component(s) remaining on the display 2, antimicrobial properties can be obtained. The cleaner disclosed herein can provide (apply) an antimicrobial component to the surface being cleaned by taking advantage of organic dirt removal work and give antimicrobial treatment to the surface being cleaned.

The portable device in this embodiment is a tablet terminal whose display is entirely formed of tempered glass such as aluminosilicate glass although it is not limited to this as described earlier.

The size of the cylindrical PSA sheet roll is not particularly limited. When it is used on portable devices such as tablet terminals, etc., its diameter (which refers to the diameter (outer diameter) before used; the same applies hereinafter) is preferably 4 mm or larger (more preferably 10 mm or larger, e.g. 15 mm or larger, typically 20 mm or larger). From the standpoint of the maneuverability and portability, the diameter is preferably 50 mm or smaller (e.g. 35 mm or smaller, typically 30 mm or smaller).

The PSA sheet constituting the PSA sheet roll preferably has cut lines (not shown in the drawings) at an interval of a length approximately equal to the circumference of the roll. The cut lines provide cutting means to efficiently refresh the PSA layer surface (outer surface of the dirt-collecting member) with a reduced cleaning (dirt-removing) ability or a reduced ability to provide antimicrobial properties to the surface to be cleaned after the cleaner is used several times. The cut lines can be, for instance, lines of long holes or wavy slits; intermittent slits such as perforation; and the like. The cut lines are preferably arranged to run across the PSA sheet in the width direction (direction perpendicular to the length direction). Refreshing the outer surface (lateral surface) of the dirt-collecting member is not limited to the cutting means. For instance, intermittent slits such as perforation can be spirally formed in a direction intersecting the winding

direction of the PSA sheet roll (typically, in a direction intersecting the width direction at an angle between 30° and 60°). Alternatively, instead of intermittent slits such as perforation, the PSA sheet constituting the PSA sheet roll may comprise slits (continuous cut lines) at a prescribed interval. In this embodiment, the PSA sheet constituting the PSA sheet roll is fully cut in advance at the prescribed intervals in the winding direction of the roll. Thus, the outer surface of the PSA sheet roll can be peeled over the length of the prescribed interval to easily refresh the outer surface.

The cleaner 10 can be produced by suitably employing heretofore known techniques. For instance, PSA sheet roll 30 of cleaner 10 can be fabricated in the same manner as conventional roller cleaners. In other words, a PSA composition is applied to the surface 36A of a long sheet of support substrate 36 by various heretofore known coating means and allowed to dry as necessary to form a PSA layer 32. As for a hot melt PSA (thermoplastic PSA), the PSA heated into a molten state can be applied as the PSA composition and the PSA is allowed to cool to near room temperature to form a PSA layer. Thus, the drying can be omitted. The PSA sheet 31 is wound around holding member 20 with the PSA layer 32 on the outside to form PSA sheet roll 30 in a form of a roll. A terminal section of grip member 40 is further attached to holding member 20 in a freely rotatable manner to construct cleaner 10. The structure of attachment of grip member 40 to holding member 20 can be similar to those in conventional roller cleaners and does not characterize the present invention. Accordingly, detailed description is omitted.

The sticky cleaner is not limited to the embodiment above. The sticky cleaner may be formed, for instance, solely with a dirt-collecting member. Examples of such a sticky cleaner include a cleaner formed solely with a dirt-collecting member in a form of a sphere, column, cylinder, hexahedron (e.g. cuboid), sheet, etc.

In the above embodiment, the dirt-collecting member is formed with the support substrate and PSA layer, but the sticky cleaner is not limited to this embodiment. For instance, the dirt-collecting member may be formed solely with a PSA (substrate-free PSA). When the dirt-collecting member has a support substrate, the shape of the support substrate is not particularly limited. For instance, the dirt-collecting member may have a PSA layer on the outer surface of a spherical support substrate.

In the above embodiment, the grip member supports the holding member in a freely rotatable manner, but the sticky cleaner is not limited to this embodiment. For instance, the grip member may be directly or indirectly joined (connected or detachably joined) to the dirt-collecting member. In an example of such a sticky cleaner, a columnar or cuboid PSA body is fastened to one end of a pole-shaped grip member. Alternatively, the grip member may have a flat portion and the dirt-collecting member may be fastened to one face of the flat portion.

The PSA included in the cleaner disclosed herein is not particularly limited in composition or in form as long as it can collect organic dirt upon contact with a surface subject to cleaning and can also provide antimicrobial properties to the surface being cleaned. For instance, the PSA may be formed from a PSA composition as an aqueous PSA composition including a water-soluble PSA composition and a water-dispersed PSA composition, or as a solvent-based PSA composition, etc. A solvent-free PSA formed from an active energy ray (e.g. UV ray) curable PSA composition or from a hot melt PSA composition can also be preferably used. To remove human sebum dirt, it is preferable to use a PSA formed from a solvent-based PSA composition or a solvent-free PSA. From the standpoint of the productivity and the handling properties of the PSA composition, a hot melt PSA is preferable.

The PSA can be, for instance, an acrylic PSA, a rubber-based PSA (e.g. a natural rubber-based PSA), a urethane-based PSA, a silicone-based PSA, etc. From the standpoint of the ease of adjusting the adhesive strength and other adhesive properties, the cost and so on, a rubber-based PSA or an acrylic PSA can be preferably used. Here, the acrylic PSA refers to a PSA that comprises an acrylic polymer as the base polymer (the primary component among the polymers, i.e. a component accounting for more than 50 % by weight). The same applies to the rubber-based and other PSAs as well. In typical, the cleaner disclosed herein can be constructed so that the same PSA works to remove organic dirt from a surface subject to cleaning as well as to provide antimicrobial properties to the same surface.

<Base Polymer>

The art disclosed herein can be preferably implemented in an embodiment where the PSA is an acrylic PSA comprising an acrylic polymer as the base polymer. The acrylic polymer can be synthesized from a starting monomer mixture that comprises, as the primary monomer, an alkyl (meth)acrylate having an alkyl group. Here, the primary monomer refers to a monomer that accounts for more than 50 % by weight of all the monomers. As used herein, the term "(meth)acrylate" comprehensively refers to acrylate and methacrylate. Similarly, the terms "(meth)acryloyl" and "(meth)acryl" comprehensively refer to acryloyl and methacryloyl and to acryl and methacryl, respectively.

When the starting monomer mixture comprises two or more species of monomers, the acrylic polymer may be a random copolymer, a block copolymer, a graft copolymer, etc. From the standpoint of the ease of production and the handling properties, preferable acrylic polymers include a random copolymer and a block copolymer. For the acrylic polymer, solely one species or a combination of two or more species can be used.

[Acrylic Random Copolymer]

The acrylic polymer according to a preferable embodiment comprises an acrylic random copolymer synthesized from a starting monomer mixture that comprises an alkyl (meth)acrylate having an alkyl group as the primary monomer. As the alkyl (meth)acrylate, for instance, a compound represented by a general formula (1) shown below can be suitably used:

$$CH_2=CR^1COOR^2 \qquad (1);$$

Here, $R^1$ in the general formula (1) is a hydrogen atom or a methyl group. $R^2$ is an alkyl group having 1 to 20 carbon atoms (hereinafter, such a range of the number of carbon atoms may be indicated as "$C_{1-20}$"). From the standpoint of the storage elastic modulus of the PSA, etc., an alkyl (meth)acrylate having a $C_{1-14}$ (e.g. $C_{1-10}$) alkyl group is preferable. The alkyl group can be acyclic (linear or branched) or may include a cyclic structure.

Examples of the alkyl (meth)acrylate having a $C_{1-20}$ alkyl group include methyl (meth)acrylate, ethyl (meth)acrylate, n-propyl (meth)acrylate, isopropyl (meth)acrylate, n-butyl (meth)acrylate, isobutyl (meth)acrylate, s-butyl (meth)acrylate, t-butyl (meth)acrylate, n-pentyl (meth)acrylate, isoamyl (meth)acrylate, neopentyl (meth)acrylate, n-hexyl (meth)acrylate, cyclohexyl (meth)acrylate, n-heptyl (meth)acrylate, n-octyl (meth)acrylate, isooctyl (meth)acrylate, 2-ethylhexyl (meth)acrylate, n-nonyl (meth)acrylate, isononyl (meth)acrylate, decyl (meth)acrylate, isodecyl (meth)acrylate, bornyl (meth)acrylate, isobornyl (meth)acrylate, undecyl (meth)acrylate, lauryl (meth)acrylate, tridecyl (meth)acrylate, tetradecyl (meth)acrylate, pentadecyl (meth)acrylate, stearyl (meth)acrylate, heptadecyl (meth)acrylate, octadecyl (meth)acrylate, nonadecyl (meth)acrylate and eicosyl (meth)acrylate. These alkyl (meth)acrylates may be used singly as one species or in a combination of two or more species.

From the standpoint of the removability of organic dirt (e.g. sebum dirt), the starting monomer mixture preferably comprises, as the primary monomer, an alkyl (meth)acrylate having an acyclic $C_{4-14}$ alkyl group. With the inclusion of an alkyl (meth)acrylate having an acyclic $C_{6-12}$ alkyl group as the primary monomer, greater organic dirt removability tends to be obtained. Favorable examples of such an alkyl (meth)acrylate include 2-ethylhexyl acrylate (2EHA), isooctyl acrylate (IOA), isononyl acrylate (INA) and lauryl methacrylate. Among them, 2EHA, IOA and INA are preferable, with 2EHA being particularly preferable.

The ratio of the primary monomer in all the monomers forming the acrylic random copolymer is preferably 60 % by weight or higher, more preferably 80 % by weight or higher, or yet more preferably 90 % by weight or higher. The maximum ratio of the primary monomer in all the monomers is not particularly limited. From the standpoint of facilitating the adjustment of the adhesive properties (adhesive strength, cohesive strength, etc.), it is usually preferably 99 % by weight or less (e.g. 98 % by weight or less, typically 95 % by weight or less). The acrylic random copolymer can be a polymerization product of essentially just the primary monomer.

For purposes of adjusting the adhesive properties, etc., the starting monomer mixture used in polymerization of the acrylic random copolymer may further comprise, in addition to the primary monomer, a secondary monomer (possibly an oligomer) copolymerizable with the primary monomer. As such a secondary monomer, a monomer having a functional group (or "functional group-containing monomer" hereinafter) can be cited. The functional group-containing monomer can be added to incorporate crosslinking points into the acrylic polymer to facilitate adjustment of the adhesive properties (adhesive strength, cohesive strength, etc.). Examples of such a functional group-containing monomer include a carboxy-group-containing monomer, an acid-anhydride-group-containing monomer, a hydroxy-group-containing monomer, an amide-group-containing monomer, an amino-group-containing monomer, an epoxy-group (glycidyl group)-containing monomers, an alkoxy-group-containing monomers, and an alkoxysilyl-group-containing monomers. These can be used singly as one species or in a combination of two or more species. Among these, for their abilities to favorably introduce crosslinking points into the acrylic polymer and also for easy adjustment of the PSA's crosslink density, preferable functional group-containing monomers include a carboxy group-containing monomer, a hydroxy group-containing monomer and an epoxy group-containing monomer, with the carboxy-group-containing monomer and the hydroxy-group-containing monomer being more preferable.

Examples of a carboxy-group-containing monomer include ethylenic unsaturated monocarboxylic acids such as acrylic acid, methacrylic acid, crotonic acid, carboxyethyl (meth)acrylate, and carboxypentyl (meth)acrylate; and ethylenic unsaturated dicarboxylic acids such as itaconic acid, maleic acid, fumaric acid, and citraconic acid. Among these, acrylic acid and/or methacrylic acid are preferable, and acrylic acid is especially preferable.

Examples of an acid-anhydride-group-containing monomers include acid anhydrides of the ethylenic unsaturated dicarboxylic acids listed above such as maleic acid anhydride, and itaconic acid anhydride.

Examples of a hydroxy-group-containing monomer include hydroxyalkyl (meth)acrylates such as 2-hydroxyethyl (meth)acrylate, 2-hydroxypropyl (meth)acrylate, 3-hydroxypropyl (meth)acrylate, 2-hydroxybutyl (meth)acrylate, and 4-hydroxybutyl (meth)acrylate; and unsaturated alcohols such as N-methylol(meth)acrylamide, vinyl alcohol, allyl alcohol, 2-hydroxyethyl vinyl ether, 4-hydroxybutyl vinyl ether, and diethylene glycol monovinyl ether.

Examples of an amide-group-containing monomer include (meth)acrylamide, N,N-dimethyl(meth)acrylamide, N-butyl(meth)acrylamide, N-methylol(meth)acrylamide, N-methylolpropane(meth)acrylamide, N-methoxymethyl(meth)acrylamide, and N-butoxymethyl(meth)acrylamide.

Examples of an amino-group-containing monomer include aminoethyl (meth)acrylate, N,N-dimethylaminoethyl (meth)acrylate, and t-butylaminoethyl (meth)acrylate.

Examples of an epoxy-group (glycidyl group)-containing monomer include glycidyl (meth)acrylate, methylglycidyl (meth)acrylate, and allyl glycidyl ether.

Examples of an alkoxy-group-containing monomer include methoxyethyl (meth)acrylate, ethoxyethyl (meth)acrylate.

Examples of an alkoxysilyl-group-containing monomer include 3-(meth)acryloxypropyltrimethoxysilane, 3-(meth)acryloxypropyltriethoxysilane, 3-(meth)acryloxypropylmethyldimethoxysilane, and 3-(meth)acryloxypropylmethyldiethoxysilane.

When a functional group-containing monomer as described above is used, the functional group-containing monomer (preferably a carboxyl group-containing monomer) is preferably added at 1 % to 10 % by weight (e.g., 2 % to 8 % by weight, typically 3 % to 7 % by weight) of all the monomers.

To increase the cohesive strength of the acrylic polymer, etc., another monomer besides the functional-group-containing monomer can be included as a secondary monomer. Examples of such a monomer include vinyl-ester-based monomers such as vinyl acetate, and vinyl propionate; and aromatic vinyl compounds such as styrene, substituted styrenes ($\alpha$-methylstyrene, etc.), and vinyl toluene.

The method for synthesizing the acrylic polymer (acrylic random copolymer) from the monomer mixture is not particularly limited. A general polymerization method heretofore known can be suitably employed, such as heretofore known solution polymerization, emulsion polymerization, bulk polymerization, and suspension polymerization. The embodiment of the polymerization is not particularly limited. It can be carried out with suitable selection of a heretofore known monomer supply method, polymerization conditions (temperature, time, pressure, etc.), and other components (polymerization initiator, surfactant, etc.) used besides the monomer(s).

The polymerization initiator is not particularly limited. Examples include an azo-based initiator such as 2,2'-azobisisobutylonitrile; a peroxide-based initiator such as benzoyl peroxide; a substituted ethane-based initiator such as phenyl-substituted ethane; and a redox-based initiator by a combination of a peroxide and a reducing agent (e.g. a combination of a peroxide and sodium ascorbate). The amount of polymerization initiator used can be suitably selected in accordance with the species of polymerization initiator, the monomer species (the composition of the monomer mixture) and so on. It is usually suitably selected from a range of, for instance, about 0.005 part to 1 part by weight to 100 parts by weight of all the monomers. The polymerization temperature can be, for example, around 20 °C to 100 °C (typically 40 °C to 80 °C).

The weight average molecular weight (Mw) of the acrylic random copolymer is not particularly limited. For instance, an acrylic random copolymer with Mw of about $30 \times 10^4$ to $100 \times 10^4$ can be favorably used as the base polymer. In a preferable embodiment, the PSA may be formed from a solvent-based PSA composition that comprises an acrylic random copolymer with Mw in this range as the base polymer.

[Acrylic Block Copolymer]

The acrylic polymer according to a preferable embodiment comprises an acrylic block copolymer having a hard segment (A) (or a "A-block" hereinafter) and a soft segment (B) (or a "B-block" hereinafter) in one molecule. In the structure of the acrylic block copolymer, the hard segment (A) refers to the relatively hard block in relation to the soft segment (B) in the acrylic copolymer. The soft segment (B) refers to the relatively soft block in relation to the hard segment (A) in the structure of the acrylic block copolymer.

The acrylic block copolymer may show characteristics of thermoplastic polymers (typically thermoplastic elastomers). The PSA disclosed herein comprises the acrylic block copolymer as the base polymer and thus may be a PSA suited for hot melt application (i.e. a hot melt PSA). The hot melt PSA is preferable from the standpoint of reducing environmental stress, etc., because the amount of organic solvents used can be reduced as compared with a general organic solvent-based acrylic PSA.

Herein, the acrylic block copolymer refers to a polymer having a block structure that comprises, as a monomer unit (monomeric component) constituting the copolymer, a monomer unit derived from a monomer having at least one (meth)acryloyl group per molecule (or an "acrylic monomer" hereinafter). In other words, it refers to a block copolymer comprising a monomer unit derived from an acrylic monomer. For instance, in a preferable acrylic block copolymer, 50 % by weight or more of all monomer units are derived from an acrylic monomer. Such an acrylic block copolymer can be preferably synthesized from starting monomer(s) comprising, as the primary monomer, an alkyl (meth)acrylate having an alkyl group.

A preferable acrylic block copolymer comprises at least one acrylate block (which hereinafter may be referred to as an Ac block) and at least one methacrylate block (which hereinafter may be referred to as an MAc block). For instance, a preferable block copolymer has a structure in which Ac blocks and MAc blocks are positioned alternately. The total block number of Ac blocks and MAc blocks comprised in one polymer molecule can be about 2.5 to 5 in average (e.g. about 2.7 to 3.3, typically about 3).

In typical, the Ac block preferably comprises an alkyl acrylate as the primary monomer. In other words, 50 % by weight or more of all monomeric units constituting the Ac block are preferably monomer units derived from an alkyl acrylate. 75 % by weight or more (e.g. 90 % by weight or more) of the monomer units can be derived from an alkyl acrylate as well. In a preferable embodiment, the Ac block in the acrylic block copolymer is a polymer essentially formed of one, two or more species (typically one species) of alkyl acrylate. Alternatively, the Ac block may be a copolymer of an alkyl acrylate and other monomer (e.g. an alkyl methacrylate, etc.).

An example of the Ac block-constituting alkyl acrylate is an alkyl acrylate having 1 to 20 (preferably 4 to 14, e.g. 6 to 12) carbon atoms in its alkyl group. Examples include methyl acrylate, ethyl acrylate, n-propyl acrylate, isopropyl acrylate, n-butyl acrylate (BA), isobutyl acrylate, tert-butyl acrylate, n-pentyl acrylate, n-hexyl acrylate, n-heptyl acrylate, n-octyl acrylate, isooctyl acrylate (IOA), 2-ethylhexyl acrylate (2EHA), n-nonyl acrylate, isononyl acrylate (INA), decyl acrylate, lauryl acrylate, and stearyl acrylate. These can be used singly as one species or in a combination of two or more species.

In a preferable embodiment, 50 % by weight or more of monomers constituting the Ac block is an alkyl acrylate having 4 to 14 carbon atoms in its alkyl group. The ratio of alkyl acrylate having 4 to 14 carbon atoms in alkyl group can be 75 % by weight or greater or can be essentially 100 % by weight (e.g. greater than 99 % by weight, but 100 % by weight or less). For instance, it is preferable to use a structure in which the monomer unit(s) constituting the Ac block essentially consist of BA or 2EHA , or comprise the two species, BA and 2EHA, and so on. The weight ratio of BA to 2EHA is not particularly limited. For instance, it can be 10/90 to 90/10, preferably 80/20 to 20/80, or more preferably 30/70 to 70/30, for example, 60/40 to 40/60.

It is typically preferable that the MAc block comprises an alkyl methacrylate as the primary monomer. Of all the monomer units constituting the MAc, 75 % by weight or more (e.g. 90 % by weight or more) can be derived from an alkyl methacrylate. In a preferable embodiment, the MAc block in the acrylic block copolymer is essentially formed of one, two or more species (typically one species) of alkyl methacrylate. Alternatively, the MAc block may be a copolymer of an alkyl methacrylate and other monomer (e.g., an alkyl acrylate).

The alkyl methacrylate constituting the MAc block may be an alkyl methacrylate whose alkyl group has 1 to 20 (preferably 1 to 14) carbon atoms. Specific examples thereof include methyl methacrylate, ethyl methacrylate, n-propyl methacrylate, isopropyl methacrylate, n-butyl methacrylate, isobutyl methacrylate, tert-butyl methacrylate, n-pentyl methacrylate, n-hexyl methacrylate, n-heptyl methacrylate, n-octyl methacrylate, isooctyl methacrylate, 2-ethylhexyl methacrylate, n-nonyl methacrylate, isononyl methacrylate, decyl methacrylate, lauryl methacrylate, stearyl methacrylate, etc. These can be used singly as one species or in a combination of two or more species.

In a preferable embodiment, 50 % by weight or more of the monomers constituting the MAc block is an alkyl methacrylate having an alkyl group with 1 to 4 (preferably 1 to 3) carbon atoms. The ratio of the alkyl methacrylate having 1 to 4 carbon atoms in its alkyl group can be 75 % by weight or greater, or essentially 100 % by weight (e.g. greater than 99 % by weight, but 100 % by weight or less). Especially preferable alkyl methacrylates include methyl methacrylate (MMA) and ethyl methacrylate (EMA). For example, the monomers preferably employed may consist essentially of MMA alone, EMA alone, both MMA and EMA, or the like.

The acrylic block copolymer may be a copolymer comprising A blocks (hard segments (A)) and B blocks (soft segments (B)) positioned alternately such as AB structure, ABA structure, ABAB structure, ABABA structure, etc., with the A block having been formed of a polymer having a rigid structure with excellent cohesive strength and elasticity, and the B block having been formed of a polymer having a flexible structure with excellent viscosity. A PSA comprising as its base polymer an acrylic block copolymer having such a structure may form a PSA layer combining cohesive strength and elasticity as well as viscosity at high levels. A PSA having such a composition can be preferably used as a hot melt PSA. An acrylic block copolymer having a structure (such as ABA structure, ABABA structure, etc.) with A blocks at both termini of the molecule can be preferably used. An acrylic block copolymer having such a structure is preferable because it is likely to have a good balance of cohesion and thermoplasticity. From the standpoint of reducing the melt viscosity, etc., an acrylic block copolymer having a linear structure is advantageous as compared to a species having a stellar structure or a branched structure.

When the acrylic block copolymer comprises two or more A blocks, the compositions, molecular weights (polymerization degrees), structures, etc., of these A blocks can be the same with or different from each other. When the acrylic block copolymer comprises two or more B blocks, the same applies to the B blocks.

As the A block, an MAc block as those described above can be preferably used. As the B block, an Ac block as those described above can be preferably used. In a preferable embodiment, the acrylic block copolymer is a triblock copolymer having a structure of MAc-Ac-MAc (ABA structure). For instance, can be preferably used a triblock copolymer

with two MAc blocks having essentially identical monomer compositions.

A preferable acrylic block copolymer in the art disclosed herein has, as the soft segment (B), an Ac block comprising an alkyl acrylate having 6 or more (e.g. 6 to 12) carbon atoms in its alkyl group. In the monomer units constituting the Ac block, the ratio of the alkyl acrylate having 6 or more carbon atoms in its alkyl group can be, for instance, 10 % by weight or higher, or it is preferably 20 % by weight or higher, more preferably 30 % by weight or higher, or yet more preferably 40 % by weight or higher.

In a preferable embodiment, as the soft segment (B), an acrylic block copolymer that has an Ac block comprising as its primary monomer the alkyl acrylate having 6 or more (e.g. 6 to 12) carbon atoms in its alkyl group can be used. In other words, it is preferable that one, two or more species of alkyl acrylate having 6 or more carbon atoms in its alkyl group account for 50 % by weight or more of the monomer units constituting the Ac block. Of the monomer units that constitute the Ac block, the ratio of alkyl acrylate having 6 or more carbon atoms in its alkyl group can be, for instance, 55 % by weight or higher, or 60 % by weight or higher. It is preferably 70 % by weight or higher, more preferably 85 % by weight or higher, yet more preferably 95 % by weight or higher, or it can be essentially 100 % by weight as well. For instance, a preferable acrylic block copolymer has, as the soft segment (B), an Ac block constituted solely with 2EHA as the monomer unit.

Preferable examples of the alkyl acrylate having 6 or more carbon atoms in its alkyl group include 2-ethylhexyl acrylate (2EHA), n-octyl acrylate, isononyl acrylate and n-hexyl acrylate.

An acrylic block copolymer that has as the soft segment (B) an Ac block comprising an alkyl acrylate having 6 or more carbon atoms in its alkyl group may have an excellent organic dirt-collecting ability. Thus, for instance, it may bring about a higher fingerprint removal rate in the fingerprint removability test described later.

The acrylic block copolymer that has as the soft segment (B) an Ac block comprising an alkyl acrylate having 6 or more carbon atoms in its alkyl group may have excellent compatibility with plasticizer. In a PSA having a composition that includes the copolymer and a plasticizer, this is preferable from the standpoint of reducing the bleeding (bleed-out) of the plasticizer. Because it allows suitable inclusion of a greater amount of plasticizer, it leads to advantages such as permitting great latitude in selecting the amount of plasticizer added and easy adjustment of the adhesive strength.

In another preferable embodiment, it is possible to use an acrylic block copolymer that has, as the soft segment (B), an Ac block formed with monomer units that comprise an alkyl acrylate whose alkyl group has 6 or more (e.g. 6 to 12, typically 6 to 9) carbon atoms and an alkyl acrylate whose alkyl group has 2 to 5 (e.g. 3 to 4, typically 4) carbon atoms at a weight ratio of 20/80 to 80/20 (more preferably 30/70 to 70/30, or yet more preferably 40/60 to 60/40, e.g. 45/55 to 55/45). Such an acrylic block copolymer may have well-balanced organic dirt removability and cohesiveness. For instance, it is preferable to use an acrylic block copolymer that has, as the soft segment (B), an Ac block formed with monomer units that comprise 2EHA and BA at an aforementioned weight ratio. The monomer units may be formed solely from 2EHA and BA.

The weight ratio of hard segment (A) to soft segment (B) is not particularly limited in the acrylic block copolymer. For instance, the weight ratio (A/B) of hard segment (A) to soft segment (B) can be in a range of 4/96 to 90/10, or it is usually suitably in a range of 7/93 to 70/30 or preferably in a range of 10/90 to 50/50 (e.g. more preferably 15/85 to 40/60, e.g. 15/85 to 25/75). In the acrylic block copolymer comprising two or more hard segments (A), the ratio of total weight of these hard segments (A) to weight of soft segment (B) is preferably in these ranges. The same applies to the acrylic block copolymer comprising two or more soft segments (B). When the ratio of hard segment (A) (e.g. MAc block) is high, the adhesive strength tends to decrease, likely providing easy-release properties. When the ratio of soft segment (B) (e.g. Ac block) is high, the organic dirt-collecting ability tends to increase.

In a preferable example of the acrylic block copolymer disclosed herein, the starting monomers corresponding to all the monomer units constituting the acrylic block copolymer comprise an alkyl (meth)acrylate (X) having 1 to 3 carbon atoms in its alkyl group and an alkyl (meth)acrylate (Y) having 6 or more (e.g. 6 to 12) carbon atoms in its alkyl group. The weight ratio (X/Y) of alkyl (meth)acrylate (X) to alkyl (meth)acrylate (Y) can be, for instance, 4/96 to 90/10. An acrylic block copolymer with the weight ratio being 7/93 to 70/30 is preferable, a species with 10/90 to 50/50 is more preferable, a species with 15/85 to 40/60 is yet more preferable, and a species with15/85 to 30/70 (e.g. 15/85 to 25/75) is particularly preferable. When the ratio of alkyl (meth)acrylate (X) is high, the adhesive strength tends to decrease, likely providing easy-release properties. When the ratio of alkyl (meth)acrylate (Y) is high, the organic dirt-collecting ability tends to increase. As the alkyl (meth)acrylate (X), an alkyl methacrylate having 1 to 3 carbon atoms in its alkyl group is preferable. As the alkyl (meth)acrylate (Y), an alkyl acrylate having 6 or more (e.g. 6 to 12) carbon atoms in its alkyl group is preferable.

In another preferable example of the acrylic block copolymer disclosed herein, the starting monomers corresponding to all the monomer units constituting the acrylic block copolymer comprise methyl methacrylate (MMA) and 2-ethylhexyl acrylate (2EHA) at a weight ratio (MMA/2EHA) of their contents of 4/96 to 90/10. An acrylic block copolymer with the weight ratio being 7/93 to 70/30 is preferable, a species with 10/90 to 60/40 is more preferable, a species with 20/80 to 50/50 is yet more preferable, and a species with 25/75 to 40/60 (e.g. 25/75 to 35/65) is particularly preferable. When the MMA content is high, the adhesive strength tends to decrease, likely providing easy-release properties. When

the 2EHA content is high, the organic dirt-collecting ability tends to increase.

The composition of monomer units constituting an acrylic block copolymer can be determined based on the results of NMR analysis. In particular, the NMR analysis can be carried out, using, for instance, AVAVCEIII-600 (with Cryo Probe) available from Bruker Biospin as the NMR system, under the conditions shown below. For instance, the weight ratio of MMA to 2EHA in the starting monomers can be determined based on the ratio of integrated intensities of peaks at 4.0 ppm (2EHA1) and 3.6 ppm (MMA1) in the $^1H$ NMR spectrum.

[NMR Analysis Conditions]

Measurement frequency: $^1H$, 600 MHz
Flip angle: 30°
Measurement solvent: $CDCl_3$
Measurement temperature: 300 K
Standard chemical shift: measurement solvent ($CDCl_3$, $^1H$: 7.25 ppm)

In the art disclosed herein, the acrylic block copolymer's Mw is not particularly limited. For instance, an acrylic block copolymer having a Mw of about $3 \times 10^4$ to $30 \times 10^4$ can be preferably used. Usually, the acrylic block copolymer has a Mw in a range of preferably about $3.5 \times 10^4$ to $25 \times 10^4$ or more preferably in a range of about $4 \times 10^4$ to $20 \times 10^4$ (e.g., $4.5 \times 10^4$ to $15 \times 10^4$). A higher Mw of the acrylic block copolymer is advantageous from the standpoint of increasing the adhesive properties (e.g. cohesion) and increasing the organic dirt-collecting ability. With increasing Mw of the acrylic block copolymer, the amount of plasticizer that can be suitably included tends to increase. On the other hand, a low Mw of the acrylic block copolymer is advantageous from the standpoint of reducing the viscosity (melt viscosity) of the PSA. It is particularly meaningful for a hot melt PSA to have lower melt viscosity.

The Mw of an acrylic block copolymer herein refers to the value based on standard polystyrene that is determined by gel permeation chromatography (GPC) with respect to a sample prepared by dissolving the copolymer in tetrahydrofuran (THF). In particular, the GPC measurement can be performed, using, for instance, trade name "HLC-8120GPC" available from Tosoh Corporation as the GPC measurement system, under the conditions shown below.

[GPC Measurement Conditions]

- Columns: available from Tosoh Corporation, TSK gel Super HZM-H / HZ4000 / HZ3000 / HZ2000
- Column size: 6.0 mm I.D. × 150 mm each
- Eluent: THF
- Flow rate: 0.6 mL/min
- Detector: differential refractometer (RI)
- Column temperature (measurement temperature): 40 °C
- Sample concentration: about 2.0 g/L (THF solution)
- Sample injection volume: 20 μL

In the acrylic block copolymer in the art disclosed herein, a monomer (other monomer) other than an alkyl acrylate and an alkyl methacrylate may be copolymerized. Examples of the other monomer include vinyl compounds having functional groups such as alkoxy group, epoxy group, hydroxy group, amino group, amide group, cyano group, carboxy group, acid anhydride group, etc.; vinyl esters such as vinyl acetate; aromatic vinyl compounds such as styrene; vinyl group-containing heterocyclic compounds such as N-vinylpyrrolidone and the like. Alternatively, it can be an alkyl acrylate having a structure with an acryloyl group coupled to a fluorinated alkyl group, a fluorinated alkyl acrylate and a fluorinated alkyl methacrylate. The other monomer may be used, for instance, to adjust the properties (adhesive properties, ease of molding, etc.) of the PSA layer and its content is suitably 20 % by weight or less (e.g. 10 % by weight or less, typically 5 % by weight or less) of all the monomers constituting the acrylic block copolymer. In a preferable embodiment, the acrylic block copolymer is essentially free of the other monomers. For instance, in a preferable acrylic block copolymer, the other monomer content is less than 1 % by weight (typically 0 to 0.5 % by weight) of all monomers or under the detection limit.

Such an acrylic block copolymer can be readily synthesized by a known method (e.g. see Japanese Patent Application Publication Nos. 2001-234146 and H11-323072), or a commercial product is readily available. Examples of the commercial product include trade name "KURARITY" series (e.g., those with product numbers LA2140e, LA2250,

etc.) available from Kuraray Co., Ltd., trade name "NABSTAR" available from Kaneka Corporation, and the like. As the method for synthesizing the acrylic block copolymer, living polymerization method can be preferably employed. According to living polymerization, while keeping the weatherability inherent in the acrylic polymer, because of the excellent structure control unique to the living polymerization, an acrylic block copolymer having excellent thermoplasticity can be synthesized. Since the molecular weight distribution can be controlled in a narrow range, insufficient cohesion caused by the presence of low molecular weight components can be reduced to obtain an easily releasable PSA (and thereby a PSA sheet (dirt-collecting member)).

In the art disclosed herein, for the acrylic block copolymer, solely one species or a combination of two or more species can be used. For instance, an acrylic block copolymer (H) that has a relatively high Mw and an acrylic block copolymer (L) that has a lower Mw than the acrylic block copolymer (H) can be used at a suitable weight ratio. By this, while curbing the increase in viscosity (melt viscosity) of the PSA, the organic dirt-collecting ability can be effectively increased. From the standpoint of obtaining greater effects of the combined use of the acrylic block copolymer (H) and acrylic block copolymer (L), they are preferable used so that the weight ratio (H/L) of acrylic block copolymer (H) to acrylic block copolymer (L) is in a range of 5/95 to 95/5 (preferably 10/90 to 90/10).

Each of the acrylic block copolymers used in such combination preferably has a Mw in a range of $3 \times 10^4$ to $30 \times 10^4$. For example, a preferable combination includes an acrylic block copolymer (H) having a Mw in a range of $5 \times 10^4$ to $20 \times 10^4$ (e.g. $7 \times 10^4$ to $20 \times 10^4$) and an acrylic block copolymer (L) having a Mw in a range of $3 \times 10^4$ to $8 \times 10^4$ and lower than the Mw of the acrylic block copolymer (H). A more preferable example of the combination includes an acrylic block copolymer (H) having a Mw in a range of $6 \times 10^4$ to $15 \times 10^4$ (e.g. $7 \times 10^4$ to $15 \times 10^4$) and an acrylic block copolymer (L) having a Mw in a range of $4 \times 10^4$ to $6 \times 10^4$ and lower than the Mw of the acrylic block copolymer (H). The weight ratio (H/L) of these acrylic block copolymers can be, for instance, 40/60 to 90/10. In a preferable embodiment, the weight ratio (H/L) can be 45/65 to 90/10, also 55/45 to 90/10, or even 65/35 to 85/15 (e.g. 75/25 to 85/15).

The inclusion (presence) of two or more species of acrylic block copolymer with different Mw values, the Mw values of the respective copolymers and their weight ratio can be assessed, for instance, through the GPC measurement described earlier.

<Antimicrobial Agent>

The PSA in the art disclosed herein further comprises an antimicrobial agent in addition to the base polymer as described above. The type of antimicrobial agent is not particularly limited. It is possible to use various antimicrobial agents capable of working to provide antimicrobial properties to the surface being cleaned when the antimicrobial agent-containing PSA is allowed to make contact with the surface being cleaned. Either an organic antimicrobial agent or an inorganic antimicrobial agent can be used, or an organic antimicrobial agent and an inorganic antimicrobial agent can be used in combination.

As the inorganic antimicrobial agent, solely one species or a combination of two or more species can be used among known inorganic antimicrobial agents such as silver, copper, zinc, tin, lead and gold. As necessary, as carriers of these inorganic antimicrobial agents, zeolite, hydroxyapatite, calcium carbonate, silica gel, aluminum calcium silicate, a polysiloxane compound, zirconium phosphate, zirconium sulfate, an ion-exchanging solid material, zinc oxide and the like can be used.

Examples of the organic antimicrobial agent include known synthetic antimicrobial agents and natural antimicrobial agents. Examples of synthetic antimicrobial agents include alcohols, phenols, aldehydes, carboxylic acids, esters, ethers, nitriles, peroxides, halogens, pyridine/quinolone derivatives, triazines, isothiazolones, imidazole/thiazole derivatives, anilides, biguanides, disulfides, thiocarbamates, surfactants, and organic metals. Examples of natural antimicrobial agents include hinokitiol, mugwort extract, aloe extract, perilla leaf extract, garlic extract, Houttuynia cordata, hemerocallis, theaceae plant extract, mustard extract, horseradish extract, and bamboo extract. The organic antimicrobial agents can be used singly as one species or in a combination of two or more species.

The art disclosed herein can be preferably implemented in an embodiment where the PSA comprises at least an organic antimicrobial agent. In general, an organic antimicrobial agent tends to likely migrate within PSA as compared to an inorganic antimicrobial agent. Thus, the inclusion of at least an organic antimicrobial agent as the antimicrobial agent in the PSA may be advantageous in view of transferring the antimicrobial component(s) to the surface being cleaned and of maintaining the effect of the cleaner to provide antimicrobial properties. The PSA may contain solely an organic antimicrobial agent as the antimicrobial agent.

In an embodiment of the art disclosed herein, as the antimicrobial agent included in the PSA, a surfactant-based organic antimicrobial agent can be preferably used. Examples of the surfactant-based organic antimicrobial agent include a cationic surfactant and an ampholytic surfactant (e.g. alkyl poly(aminoethyl glycine) hydrochloride, sodium alkyl diaminoethyl glycine, etc.) capable of working as organic antimicrobial agents. The art disclosed herein can be preferably implemented in an embodiment using a cationic surfactant as the organic antimicrobial agent. For instance, a compound (a quaternary ammonium salt) represented by a general formula (2) shown below can be preferably used.

[Chem 1]

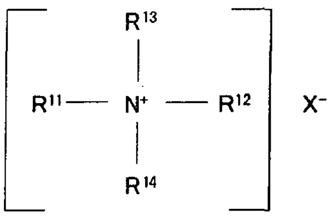

(2)

Here, $R^{11}$ to $R^{14}$ in the general formula (2) are individually selected from hydrocarbon groups with 1 to 18 carbon atoms. For instance, it is preferable that $R^{11}$ is an alkyl, aryl, or arylalkyl group with 8 to 18 carbon atoms; $R^{12}$ is an alkyl group with 1 to 18 carbon atoms; and $R^{13}$ and $R^{14}$ are alkyl groups with 1 to 4 carbon atoms. $R^{11}$ to $R^{14}$ can be the same or different.

$X^-$ in the general formula (2) is a counter ion and can be either an organic anion or an inorganic anion. Examples of the organic anion include methanesulfonate anion, trifluoromethanesulfonate anion, nonafluorobutanesulfonate anion, and p-toluenesulfonate anion. Examples of the inorganic anion include halide ions such as fluoride ion, chloride ion and bromide ion, hydroxy ion, nitrate ion, sulfate ion, tetrafluoroborate ion and hexafluorophosphate ion.

Specific examples of the compound represented by the general formula (2) include didecyldimethylammonium trifluoromethanesulfonate, didecyldimethylammonium chloride, dodecyldimethylammonium tetrafluoroborate, and benzalkonium chloride. A compound that has a divalent anion for $X^-$ in the general formula (2) and two quaternary ammonium cations as its counter ions can also be used as the organic antimicrobial agent. An example of such a compound is didecyldimethylammonium adipate. Alternatively, besides the compound represented by the general formula (2), it is also possible to use other cationic organic antimicrobial agents (typically quaternary ammonium salt-based organic antimicrobial agents), for instance, hexadecylpyridinium chloride. The art disclosed herein can be preferably implemented in an embodiment using didecyldimethylammonium trifluoromethanesulfonate as the antimicrobial agent.

While no particular limitations are imposed, from the standpoint of maintaining the antimicrobial effect after transferred to the surface being cleaned, an antimicrobial agent (typically an organic antimicrobial agent) with low volatility can be preferably used. When the PSA is formed using an antimicrobial agent-containing PSA composition comprising adhesive components in a volatile medium, such as a solvent-based PSA composition and a water-dispersed PSA composition, or formed using an antimicrobial agent-containing hot melt PSA composition, it is particularly meaningful to select a species with low volatility as the antimicrobial agent.

While no particular limitations are imposed, as the antimicrobial agent (typically an organic antimicrobial agent), it is preferable to use a species that starts decomposing at a temperature of 150 °C or above (more preferably 180 °C or above, yet more preferably 200 °C or above, e.g. 230 °C or above). The decomposition starting temperature can be obtained at a heating rate of 10 °C/min by differential scanning calorimetry (DSC). When the PSA is formed using an antimicrobial agent-containing PSA composition comprising adhesive components in a volatile medium, such as a solvent-based PSA composition and a water-dispersed PSA composition, or formed using an antimicrobial agent-containing hot melt PSA composition, it is particularly significant to select such a highly heat-resistant (thermally stable) antimicrobial agent.

The antimicrobial agent content in the PSA is not particularly limited. The antimicrobial agent content can be set so that antimicrobial properties can be provided to an article surface (e.g. a smooth glass surface) when the PSA is allowed to make contact with the surface (typically, not in a mode of contact that involves rubbing of the PSA in a planar direction on the article surface, but in a mode of contact that causes the PSA to be lightly press-bonded to or rotated on the article surface). In other words, the antimicrobial agent content can be set so that upon the contact, the antimicrobial agent is transferred from the PSA to the article surface in an amount sufficient to provide antimicrobial properties to the article surface (the surface subject to cleaning). The level of antimicrobial properties provided to the article surface upon the contact can be evaluated, for instance, by the antimicrobial activity value (R) described later in Examples. The antimicrobial agent content in the PSA is preferably set to an amount capable of providing the surface being cleaned with antimicrobial properties that give an R value of 1.0 or higher (more preferably 2.0 or higher, or yet more preferably 3.0 or higher) with respect to at least either one (preferably each) of Staphylococcus aureus and E. coli.

While no particular limitations are imposed, the antimicrobial agent content (concentration) in the PSA can be 0.5 % by weight or more of the PSA; it is usually suitably 1 % by weight or more (typically more than 1 % by weight), preferably 2 % by weight or more, more preferably 3 % by weight or more, or yet more preferably 5 % by weight or more (typically more than 5 % by weight). With increasing concentration of antimicrobial agent, greater antimicrobial properties tend to be provided to the surface being cleaned. In addition, because the amount of antimicrobial agent that can be supplied from the inner PSA to the surface of the PSA increases, the ability to maintain the function to provide antimicrobial

properties to the surface being cleaned (e.g. the ability to keep providing antimicrobial properties upon repeated use) tends to increase as well. The art disclosed herein can also be preferably implemented in an embodiment where the concentration of antimicrobial agent in the PSA is 8 % by weight or higher (even 10 % by weight or higher, e.g. higher than 10 % by weight). The maximum concentration of antimicrobial agent in the PSA is not particularly limited. From the standpoint of the raw material cost, the ease of production, etc., the concentration of antimicrobial agent is usually suitably 50 % by weight or lower, preferably 30 % by weight or lower, or yet more preferably 20 % by weight or lower. The art disclosed herein can also be preferably implemented, for instance, in an embodiment where the concentration of antimicrobial agent in the PSA is 15 % by weight or lower (e.g. 10 % by weight or lower, typically lower than 10 % by weight).

The cleaner disclosed herein can be configured so that, by adding an antimicrobial agent to other constituent (preferably, a member formed of a synthetic resin) besides the PSA or to a material forming an accessory, the constituent or the accessory itself shows antimicrobial properties. For instance, in the configuration shown in Fig. 1, the antimicrobial agent can be included in the material forming a member such as a holding member (roll core) 20, a support substrate 36, and a handle 42. Examples of the accessory include a casing constructed to be able to house a PSA sheet roll 30 when the cleaner is not in use. For the antimicrobial agent used in such a constituent or an accessory, a suitable species can be selected among known organic antimicrobial agents and inorganic antimicrobial agents as described earlier. The amount of antimicrobial agent used can be selected so that the constituent or the accessory shows suitable antimicrobial properties. For instance, it is preferably set to an amount capable of providing antimicrobial properties that give an antimicrobial activity value (R) of 0.5 or higher (more preferably 1.0 or higher, yet more preferably 2.0 or higher, or particularly preferably 3.0 or higher) with respect to at least either one (preferably each) of Staphylococcus aureus and E. coli, in a test for antimicrobial properties based on JIS Z 2801 "Antimicrobial products-Test for antimicrobial activity and efficacy." In the PSA disclosed herein, usually, the PSA itself shows antimicrobial properties (typically, a higher level of antimicrobial properties than the level of antimicrobial properties that can be provided to the surface being cleaned upon contact with the PSA).

<Plasticizer>

The PSA in the art disclosed herein preferably comprises a plasticizer. The inclusion of plasticizer in the PSA increases the ease of release. It also lowers the melt viscosity of the PSA and thus the application is further facilitated. In yet another aspect, the inclusion of plasticizer also increases the organic dirt-collecting ability on the PSA surface. This can bring about, for instance, greater sebum dirt removability.

With the inclusion of plasticizer in the PSA, the organic dirt collected on the PSA surface can be absorbed into the PSA (e.g. PSA layer) and dispersed therein. Thus, even when the dirt-collecting ability is reduced by repeated use, the dirt-collecting ability may recover in relatively short time (e.g. several minutes or several hours) as a unique dirt-collecting ability recovery effect.

The dirt-collecting ability recovery effect is described with reference to Fig. 5. As schematically illustrated in Fig. 5, when the PSA layer 32 in dirt-collecting member (PSA sheet) 31 is allowed to make contact with a surface 2 of an article 1 such as a portable device, etc., the PSA layer 32 collects organic dirt 50 stuck on the surface 2. The PSA layer 32 has a property to not only collect organic dirt 50, but also causes it to migrate into the layer. Thus, the organic dirt 50 stuck on the surface of PSA layer 32 migrates with time into the PSA layer 32, leading to a lower presence of organic dirt 50 on the surface of PSA layer 32; and eventually, the surface of PSA layer 32 will be essentially free of the presence of organic dirt 50. In other words, the surface regains the same state as before the sticky cleaner is used. Accordingly, the term "recovery effect" refers to an effect such that when the PSA collects organic dirt and the dirt-collecting ability is temporarily reduced, the dirt-collecting ability recovers in a prescribed time period (e.g. several minutes, preferably several hours) and the PSA (e.g. PSA layer) regains the ability to collect dirt. It encompasses that the dirt-collecting ability requires short time for recovery.

Examples of plasticizer include phthalic acid esters such as dioctyl phthalate, diisononyl phthalate, diisodecyl phthalate, dibutyl phthalate, etc.; adipic acid esters such as dioctyl adipate, diisononyl adipate, etc.; trimellitic acid esters such as trioctyl trimellitate, etc.; sebacic acid esters; epoxidized vegetable oils such as epoxidized soybean oil and epoxidized flax seed oil; epoxidized fatty acid alkyl esters such as epoxidized fatty acid octyl esters; cyclic fatty acid esters such as sorbitan monolaurate, sorbitan monostearate, sorbitan monooleate, sorbitan trioleate and their ethylene oxide adducts as well as their derivatives; and the like. These can be used singly as one species or in a combination of two or more species. Preferable examples of plasticizer among them include adipic acid esters, epoxidized vegetable oils and epoxidized fatty acid alkyl esters. In particular, adipic acid esters are preferable.

Such a plasticizer may work, for instance in an acrylic PSA, at a high level in decreasing the adhesive strength of the PSA and in increasing the ability to collect organic dirt. These effects can be better produced with the inclusion of the plasticizer in an acrylic PSA whose base polymer is an acrylic block copolymer. It is particularly preferable that the plasticizer is included in an acrylic PSA that comprises, as the base polymer, an acrylic block copolymer that has

as the soft segment (B) an Ac block comprising an alkyl acrylate with 6 or more (e.g. 6 to 12) carbon atoms in its alkyl group.

The amount of plasticizer added is not particularly limited. From the standpoint of providing easy-release properties, increasing the dirt-collecting ability, etc., the amount of plasticizer added to 100 parts by weight of the base polymer (e.g. an acrylic polymer) is, for instance, suitably 1 part by weight or greater. Its amount added is preferably 5 parts by weight or greater, more preferably 10 parts by weight or greater, yet more preferably 15 parts by weight or greater, or particularly preferably 20 parts by weight or greater.

Here, plasticizer generally has a low molecular weight; the higher the content is, the more likely it bleeds out to the surface of the PSA. Thus, for the purpose of transferring the antimicrobial component(s) in the PSA to the surface being cleaned so as to provide antimicrobial properties to the surface, it may be thought that the higher the plasticizer content is in the PSA, the more advantageous it is. However, the present inventor has found that the inclusion of plasticizer in the PSA would rather impair the effect of providing antimicrobial properties (especially against E. coli) to the surface being cleaned (see the working examples described later). Therefore, in order to obtain the effects of the plasticizer such as to provide easy-release nature and to increase the dirt-collecting ability as well as the effect of the antimicrobial agent to provide antimicrobial properties to the surface being cleaned while combining these effects at a high level, studies need to be conducted based on an idea different from the PSA having an antimicrobial agent-free composition.

From the standpoint of suitably obtaining the effects of the antimicrobial agent, the plasticizer content in the PSA disclosed herein is preferably 100 parts by weight or less, more preferably 80 parts by weight or less, or yet more preferably 60 parts by weight or less, relative to 100 parts by weight of the base polymer. For instance, in a PSA that comprises as the base polymer an acrylic block copolymer that has as the soft segment (B) an Ac block comprising an alkyl acrylate having 6 or more (e.g. 6 to 12) carbon atoms in its alkyl group (e.g. an Ac block comprising the alkyl acrylate as the primary monomer), the amount of plasticizer added to 100 parts by weight of the base polymer can be 5 to 100 parts by weight (preferably 10 to 70 parts by weight, more preferably 20 to 60 parts by weight, e.g. 20 to 50 parts by weight). In a preferable embodiment, the plasticizer content can be 40 parts by weight or less.

When the PSA comprises a plasticizer, the antimicrobial agent content (Wa, by weight) in the PSA is preferably set in relation to the plasticizer content (Wp, by weight). While no particular limitations are imposed, the amounts of antimicrobial agent and plasticizer can be selected so that Wa/Wp is 0.05 or higher. Wa/Wp is usually suitably 0.10 or higher, preferably 0.15 or higher, or more preferably 0.20 or higher (e.g. 0.25 or higher). The art disclosed herein can also be preferably implemented in an embodiment where Wa/Wp is 0.30 or higher. When the Wa/Wp value increases, greater antimicrobial properties tend to be provided to the surface being cleaned. The upper limit of Wa/Wp is not particularly limited. Usually, Wa/Wp is suitably 1.0 or lower, or preferably 0.80 or lower (e.g. 0.70 or lower).

While no particular limitations are imposed, from the standpoint of obtaining organic dirt removability, easy-release nature and the ability to provide antimicrobial properties to the surface being cleaned in a well-balanced manner, the total amount of antimicrobial agent and plasticizer in the PSA can be, for instance, 10 parts to 120 parts by weight, usually suitably 15 parts to 100 parts by weight, or preferably 20 parts to 80 parts by weight, relative to 100 parts of the base polymer.

<Tackifier>

The PSA in the art disclosed herein may include a tackifier as necessary. The addition of the tackifier may be beneficial in increasing the thermoplasticity (e.g. reducing the melt viscosity) of the PSA. As the tackifier, tackifier resins commonly known in the field of acrylic PSA and the like can be used. Examples include a hydrocarbon-based tackifier resin, terpene-based tackifier resin, rosin-based tackifier resin, phenolic tackifier resin, epoxy-based tackifier resin, polyamide-based tackifier resin, elastomer-based tackifier resin and ketone-based tackifier resin. These can be used singly as one species or in a combination of two or more species.

Examples of the hydrocarbon-based tackifier resin include various hydrocarbon-based resins such as aliphatic hydrocarbon resins, aromatic hydrocarbon resins (xylene resins, etc.), alicyclic hydrocarbon resins, aliphatic-aromatic petroleum resins (styrene-olefin-based copolymers, etc.), aliphatic-alicyclic petroleum resins, hydrogenated hydrocarbon resins, coumarone-based resins, coumarone-indene-based resins, and the like. Examples of the terpene-based tackifier resin include terpene-based resins such as $\alpha$-pinene polymers, $\beta$-pinene polymers, etc.; modified terpene-based resins (e.g. terpenophenolic resins, styrene-modified terpene-based resins, hydrogenated terpene-based resins, hydrogenated terpenophenolic resins, etc.) obtained by subjecting these terpene-based resins to modification (phenol modification, aromatic modification, hydrogenation modification, etc.); and so on. Examples of the rosin-based tackifier resin include unmodified rosins (raw rosins) such as gum rosin, wood rosin, etc.; modified rosins (hydrogenated rosins, disproportioned rosins, polymerized rosins, other chemically modified rosins, etc.) obtained by subjecting these unmodified rosins to hydrogenation, disproportionation, polymerization, etc.; various other rosin derivatives; and so on. Examples of phenolic tackifier resins include resol-type and novolac-type alkylphenolic resins. Preferable tackifiers among these are terpene-based resins, modified terpene-based resins and alkylphenolic resins.

The softening point of the tackifier is not particularly limited. From the standpoint of reducing the melt viscosity,

it is preferably 160 °C or lower, or more preferably 140 °C or lower. From the standpoint of avoiding an excessive increase in adhesive strength, it is preferably 60 °C or higher, or more preferably 80 °C or higher. The softening point of the tackifier is determined based on the softening point test method (ring and ball method) specified in JIS K 2207.

The amount of tackifier added is not particularly limited. From the standpoint of avoiding an excessive increase in adhesive strength, for instance, the amount of tackifier added can be, for instance, 50 parts by mass or less to 100 parts by mass of the base polymer (acrylic block copolymer), or it is usually suitably 40 parts by mass or less, or preferably 30 parts by mass or less. Alternatively, the PSA may be essentially free of such a tackifier. From the standpoint of obtaining greater effects of the tackifier (e.g. the effect to reduce the melt viscosity), the amount of tackifier added to 100 parts by mass of the base polymer is, for instance, suitably 1 part by mass or greater, preferably 5 parts by weight or greater, or more preferably 10 parts by weight or greater (e.g. 12 parts by weight or greater). The amount of tackifier added to 100 parts by weight of the base polymer can also be 15 parts by weight or greater.

<Other Components>

In addition to the base polymer, the PSA in the art disclosed herein may comprise, as necessary, another polymer or oligomer (or an optional polymer hereinafter) other than the base polymer, for instance, for purposes of adjusting the viscosity (e.g. reducing the melt viscosity) of the PSA, controlling the adhesive properties (e.g. reducing the adhesive strength) and so on. For instance, in a PSA comprising an acrylic block copolymer as the base polymer, an acrylic random copolymer having a Mw of about 500 to 10000 (typically about 800 to 5000) can be used as the optional polymer.

The amount of the optional polymer added is not particularly limited. Usually, of the total polymer content in the PSA, the optional polymer accounts for suitably 30 % by weight or less, preferably 10 % by weight or less, or more preferably 5 % by weight or less.

In a preferable embodiment, the PSA may be essentially free of a polymer other than the base polymer. The polymer content excluding the base polymer can be less than 1 part by weight (typically 0 to 0.5 part by weight) to 100 parts by weight of the base polymer.

The PSA may be crosslinked as necessary. For instance, in a PSA whose base polymer is an acrylic random copolymer, the PSA is preferably crosslinked. For the crosslinking, a known crosslinking agent can be used. Preferable examples of the crosslinking agent include organic metal salts such as zinc stearate and barium stearate, epoxy-based crosslinking agents, and isocyanate-based crosslinking agents. Oxazoline-based crosslinking agents, aziridine-based crosslinking agents, metal chelate-based crosslinking agents, melamine-based crosslinking agents and the like can be used as well. For the crosslinking agent, solely one species or a combination of two or more species can be used. In particular, for the abilities to preferably undergo crosslinking with the carboxy group and to easily provide good workability (typically easy-release nature) and further for the excellent acid resistance, epoxy-based crosslinking agents and isocyanate-based crosslinking agents are preferable; it is particularly preferable to use an epoxy-based crosslinking agent and an isocyanate-based crosslinking agent together.

The amount of the crosslinking agent used is not particularly limited. From the standpoint of the easy-release nature, to 100 parts by weight of the base polymer (e.g. an acrylic polymer), it can be about 0.01 part to 10 parts by weight (e.g. 0.05 part to 5 parts by weight, typically 0.1 part to 5 parts by weight). When an epoxy-based crosslinking agent ($C_E$) and an isocyanate-based crosslinking agent ($C_I$) are used together, their weight ratio ($C_E/C_I$) is preferably 0.01 to 1 (e.g. 0.05 to 0.5, typically 0.1 to 0.4).

The PSA in the art disclosed herein can include, as necessary, various additives known in the PSA field as well. Examples of such additives include chain transfer agent, anti-aging agent, antioxidant, UV ray absorber, photostabilizer, antistatic agent, colorant (pigment, dye, etc.) and so on. The types and amounts of these non-essential additives can be the same as usual types and amounts in this type of PSA.

When the PSA is formed as a PSA layer placed on a support substrate, the formation method is not particularly limited. For instance, it is preferable to apply a hot melt coating method where a PSA (thermoplastic PSA) heated into a molten state is directly applied to a support substrate by a heretofore known application means such as a roll coater, die coater, gravure coater, etc.; and the PSA is allowed to cool to near room temperature to form a PSA layer. In this case, the PSA is typically provided to the substrate, as a PSA essentially free of an organic solvent (i.e. solvent-free PSA). The PSA can be crosslinked by a suitable means after provided to the support substrate. Alternatively, it can be used as a non-crosslinked PSA (i.e. thermoplastic PSA) without applying a special crosslinking means. This is preferable from the standpoint of the convenience, etc.

The PSA layer is typically formed in a continuous form, but is not limited to such a form. For instance, the PSA layer may be formed in a regular or random pattern of dots, stripes, etc.

Although no particular limitations are imposed, the PSA disclosed herein suitably has a melt viscosity at 180 °C of 200 Pa·s or lower, preferably 100 Pa·s or lower, or more preferably 50 Pa·s or lower (typically 20 Pa·s or lower, e.g. 10 Pa·s or lower). Such a PSA is suited for hot melt coating. The lower limit of the melt viscosity at 180 °C is not

particularly limited. In view of the balance between the ease of application and adhesive properties, it is usually suitably 0.1 Pa·s or higher, preferably 1 Pa·s or higher, for instance, preferably 5 Pa·s or higher.

Herein, the melt viscosity can be measured by the following melt viscosity measurement method.

[Melt Viscosity Measurement Method]

Measurement device: programmable viscometer (DV-II+Pro) available from Brookfield Engineering
Measurement conditions: measurement temperature 180 °C, speed of rotation 2.5 rpm, spindle SC4-27
Measurement procedure: 14 g of PSA is placed in a sample chamber and heated to melt at 180 °C. The spindle is immersed in the molten PSA and rotated. A melt viscosity reading is taken at 30 minutes from the start of the rotation.

The PSA disclosed herein may be formed as a PSA layer upon application (typically at room temperature) as an aqueous PSA composition or as a solvent-based PSA composition to a support substrate followed by drying of the applied composition. For the solvent-based PSA composition, a general organic solvent can be used, such as ethyl acetate, toluene, hexane and ethanol. For the organic solvent, solely one species or a mixture of two or more species can be used. While no particular limitations are imposed, the PSA composition may comprise about 30 % to 70 % solid content (non-volatiles). Instead of direct application of the PSA composition to the support substrate, the PSA layer may be placed on the support substrate by applying the composition to a releasable surface (a release face), allowing it to dry to form a PSA layer on the release face, and adhering the PSA layer to a non-releasable face of the support substrate.

The thickness of the PSA layer can be suitably selected in accordance with the purpose and is not particularly limited. From the standpoint of making sufficient use of the dirt-collecting ability, of enhancing the retention of the ability to provide antimicrobial properties to the surface to be cleaned, or of increasing the recovery of the dirt-collecting ability, the thickness of the PSA layer is preferably about 10 μm or larger (e.g. 20 μm or larger, preferably 30 μm or larger, typically 40 μm or larger). When it is important to reduce the weight or size, etc., the thickness of the PSA layer is preferably 300 μm or smaller (e.g. 100 μm or smaller, preferably 70 μm or smaller, typically 60 μm or smaller). The PSA layer may be formed entirely over one face of the support substrate. Alternatively, for instance, a non-sticky region (dry edge) free of the PSA layer may be included along each edge of the support substrate's width direction.

When the dirt-collecting member disclosed herein comprises a support substrate as in this embodiment, as the support substrate, materials formed with various types of synthetic resin, non-woven fabric or paper can be used. The material of the support substrate can be a fabric, rubber sheet, foam sheet, metal foil, a composite of these, etc.

Examples of synthetic resin include a polyolefin (polyethylene, polypropylene, ethylene-propylene copolymers, etc.), polyester (polyethylene terephthalate, etc.), vinyl chloride resin, vinyl acetate resin, polyimide resin, polyamide resin, fluorocarbon resin, and the like. In particular, a support substrate made of polyethylene terephthalate (PET) can be preferably used. Examples of paper include Japanese paper (washi), kraft paper, glassine paper, high-grade paper, synthetic paper, top-coated paper, and the like. Examples of a fabric include a woven fabric and a non-woven fabric of a single species or a blend, etc., of various fibrous substances. Examples of the fibrous substance include cotton, staple fiber, Manila hemp, pulp, rayon, acetate fiber, polyester fiber, polyvinyl alcohol fiber, and polyamide fiber, polyolefin fiber. Examples of a rubber sheet include a natural rubber sheet, and a butyl rubber sheet. Examples of a foam sheet include a polyurethane foam sheet, a polyolefin foam sheet (e.g. a polyethylene foam sheet), and a polychloroprene rubber foam sheet. Examples of metal foil include aluminum foil and copper foil. The support substrate may contain as necessary various additives such as filler (inorganic filler, organic filler, etc.), anti-aging agent, antioxidant, UV ray absorber, photostabilizer, anti-static agent, lubricant, plasticizer, and colorant (pigment, dye, etc.).

When the dirt-collecting member is a single-faced PSA sheet having a PSA layer formed on a single face of a support substrate, the back face (PSA layer-free face) of the support substrate is preferably subjected to a surface treatment such as coating of a silicone-based release agent and the like to adjust the unwinding force of the PSA sheet roll to a suitable range (typically a release treatment to prevent the unwinding force from becoming excessively high).

The thickness of the support substrate can be suitably selected in accordance with the purpose and is not particularly limited. In general, the thickness is preferably about 20 μm or larger (e.g. 30 μm or larger, typically 35 μm or larger), but suitably about 200 μm or smaller (more preferably 100 μm or smaller, typically 70 μm or smaller, e.g. 50 μm or smaller).

In the dirt-collecting member (e.g. a PSA sheet), the part that makes contact with the surface to be cleaned, e.g. PSA layer-side surface of the PSA sheet, may exhibit an adhesive strength of, for instance, 2 N/25mm or less (typically 1 N/25mm or less). The adhesive strength is preferably less than 1 N/25mm. This means that the dirt-collecting member is easy-release. A cleaner having such an easy-release dirt-collecting member provides excellent dirt-removing workability since it requires little force to do the dirt-removing work on the surface. More specifically, the cleaner can be moved more smoothly on the surface (surface to be cleaned) of an article. For instance, it has advantages such that the

cleaner can be easily separated from the surface after the dirt-removing operation. Even when the article surface (e.g. display of a tablet terminal) is covered with removable protection film (e.g. protection film made of a silicone-based or polyester-based synthetic resin, etc.), because of the easy-release nature, the protection film is less likely to peel off the article while cleaning the article surface covered with the protection film (i.e. the surface of the protection film). Thus, there is an advantage that the cleaning can be easily carried out while keeping the article surface covered with the protection film. In this case, the surface to be cleaned is the protection film surface and such a surface is included in the concept of the article surface referred to herein.

From the standpoint of the dirt-removing workability, the adhesive strength is, as described earlier, preferably less than 1 N/25mm, more preferably 0.80 N/25mm or less, or yet more preferably 0.60 N/25mm or less. From the standpoint of the maneuverability in cleaning protective film surfaces, etc., the adhesive strength is suitably 0.50 N/25mm or less, preferably 0.30 N/25mm or less, or more preferably 0.20 N/25mm or less. The adhesive strength can also be less than 0.10 N/25mm.

From the standpoint of the dirt-collecting ability, the adhesive strength is usually suitably 0.001 N/25mm or greater (typically 0.005 N/25mm or greater), preferably 0.008 N/25mm or greater, or more preferably 0.01 N/25mm or greater. The adhesive strength can also be 0.03 N/25mm or greater. The art disclosed herein can be preferably implemented, for instance, in an embodiment where the adhesive strength is 0.01 N/25mm to 0.02 N/25mm.

The adhesive strength can be adjusted, for instance, by the composition of the base polymer of the PSA, the presence of crosslinking in the PSA and the crosslinking density if any, the use of plasticizer and its amount used if any, the pattern of the PSA layer formed, etc.

Herein, the adhesive strength refers to the 180° peel strength measured based on the following 180° peel test, using a stainless steel (SUS) plate as the adherend.

[180° Peel Test]

(1) As the test plate (adherend), a SUS304 steel plate polished with water-resistant sandpaper is used. The dimensions of the test plate is 2 mm or greater in thickness, about 50 mm in width and about 125 mm in length. The test plate is polished over its entire length evenly in the length direction with #360 water-resistant sandpaper.
(2) Before the adhesive strength measurement, the test plate polished with the water-resistant sandpaper is cleaned. In the cleaning procedure, the surface of the test plate is wiped with a wipe wet with reagent-grade toluene and further wiped vigorously with a dry wipe until the surface of the test plate dries out. Such cleaning procedure is repeated three times or more until the surface of the test plate is visually considered clean.
(3) The cleaned test plate (SUS plate) is left standing at a temperature of 23 ± 2 °C and 50 % relative humidity (RH) for five minutes or more and then used for the adhesive strength measurement.
(4) The dirt-collecting member (typically a PSA sheet) is cut into a rectangular sheet to obtain a test piece. The test piece is preferably about 100 mm to 300 mm long and about 15 mm to 30 mm wide. When the width is not 25 mm, the 180° peel strength (N/25mm) is determined (converted) based on the ratio of actual width to 25 mm. The thickness of the test piece is not particularly limited.
(5) The resulting test piece is applied over its adhesive face (e.g. PSAlayer-side surface) to the test plate (SUS plate) with a 2 kg roller moved back and forth once. When the test piece is adhesive over each face such as in a double-faced PSA sheet, it is preferable to apply about 25 μm thick polyethylene terephthalate (PET) film for backing to the surface opposite from the measured face.
(6) The resultant is stored in an environment at 23 °C and 50 % RH for 30 minutes. Then, using a tensile tester, based on JIS Z 0237, in an environment at 23 °C and 50 % RH, 180° peel strength to the SUS plate (to-SUS 180° peel strength) (N/25mm) is measured at a peel angle of 180°, at a tensile speed of 1000 mm/min. The tensile tester is not particularly limited. A heretofore known tensile tester can be used. For instance, measurements can be made with trade name "TENSILON" available from Shimadzu Corporation.

In the dirt-collecting member (e.g. a PSA sheet) disclosed herein, the part that makes contact with the surface to be cleaned, e.g. PSA layer-side surface of the PSA sheet, may have, but not particularly limited to, a 180° peel strength to glass of less than 1 N/25mm (more preferably 0.80 N/25mm or less, yet more preferably 0.60 N/25mm or less). A cleaner having such an easy-release dirt-collecting member provides excellent dirt-removing workability. The adhesive strength is suitably 0.50 N/25mm or less, preferably 0.30 N/25mm or less, more preferably 0.20 N/25mm or less, or particularly preferably less than 0.10 N/25mm. From the standpoint of the dirt-collecting ability, the adhesive strength is usually suitably 0.001 N/25mm or greater (typically 0.005 N/25mm or greater), preferably 0.01 N/25mm or greater, more preferably 0.02 N/25mm or greater, or yet more preferably 0.03 N/25mm or greater. The 180° peel strength to the glass plate (180° peel strength to glass) can be measured similarly to the measurement of the 180° peel strength to SUS

except that the glass plate (e.g. a commercial float glass plate) is used as the adherend.

In the dirt-collecting member (e.g. a PSA sheet) disclosed herein, the part that makes contact with the surface to be cleaned, e.g. PSA layer-side surface of the PSA sheet, may have, but not particularly limited to, a 180° peel strength to polyethylene terephthalate (PET) film of less than 1 N/25mm (more preferably 0.80 N/25mm or less, or yet more preferably 0.60 N/25mm or less). A cleaner having such an easy-release dirt-collecting member provides excellent dirt-removing workability. From the standpoint of the maneuverability in cleaning protective film surfaces, etc., the adhesive strength is suitably 0.50 N/25mm or less, preferably 0.30 N/25mm or less, more preferably 0.20 N/25mm or less, or particularly preferably less than 0.10 N/25mm. From the standpoint of the dirt-collecting ability, the adhesive strength is usually suitably 0.001 N/25mm or greater (typically 0.005 N/25mm or greater), preferably 0.01 N/25mm or greater, more preferably 0.02 N/25mm or greater, or yet more preferably 0.03 N/25mm or greater. The 180° peel strength to PET film (180° peel strength to PET) can be measured similarly to the measurement of the 180° peel strength to SUS except that PET film is used as the adherend.

When the cleaner disclosed herein includes a PSA sheet roll, the PSA sheet roll is preferably configured to deter rail drawing. Herein, the rail drawing refers to a phenomenon such that when the sheet roll is rolled (rotated) in the reverse winding direction of the sheet roll (typically in a direction in which the wound single-faced PSA sheet is peeled) on the surface being cleaned, a band of the PSA sheet is left stuck on the surface being cleaned, beginning from the outer circumferential end of the roll. Reduced occurrence of rail drawing allows for stress-free, smooth rotation of the roll on the surface being cleaned, leading to great usability. In addition, it can also prevent wasteful use of the PSA sheet due to the occurrence of rail drawing (i.e. wasting the sheet by unintentional adhesion of the PSA sheet to the surface).

For instance, in the PSA sheet, the adhesive strength (e.g. the value measured based on the 180° peel test) and the unwinding force are preferably at a balance so as to inhibit the occurrence of rail drawing on the surface (e.g. a surface made of glass such as aluminosilicate glass or a synthetic resin) being cleaned. The unwinding force herein refers to the force required to pull out the PSA sheet from the PSA sheet roll (i.e. resistive force against unwinding). The unwinding force can be perceived as the adhesive strength to the back face of the PSA sheet (typically the back face of the support substrate). For instance, when the unwinding force is excessively low as compared with the adhesive strength, if the PSA sheet roll is rotated on the surface being cleaned, the unwinding force may succumb to the adhesive strength between the PSA sheet (typically the PSA layer) and the surface being cleaned to cause rail drawing. On the other hand, an excessively high unwinding force tends to result in unsmooth unwinding of the PSA sheet.

The unwinding force can be assessed as follows. In particular, the PSA sheet roll is set in a prescribed tensile tester. In an environment at a temperature of 23 °C and 50 % RH, the outer circumferential end of the wound PSA sheet is mounted to the chuck of the tester and pulled at a rate of 300 mm/min to unwind the PSA sheet roll in the tangential direction. The unwinding force during this can be converted to and determined as the value per width (e.g. 150 mm) of PSA layer of the PSA sheet (N/150mm). For instance, a preferable PSA sheet roll has an unwinding force of about 0.5 N/150mm to 2.5 N/150mm.

[Examples]

Several working examples related to the present invention are described below although the present invention is not to be limited to these specific examples. In the following explanation, the terms "parts" and "%" are by weight unless specifically stated otherwise.

[Acrylic Polymer]

In the examples shown below, the following acrylic polymers A and B synthesized by a known living anionic polymerization method were used.

(Acrylic Polymer A)

As the acrylic polymer A, was used an acrylic block copolymer having a triblock structure of polyMAA block-poly2EHA/BA block-polyMMA block (or "MMA-2EHA/BA-MAA" hereinafter). The 2EHA to BA weight ratio (i.e. copolymerization ratio by weight) in the poly(2EHA/BA) block was 50/50. In the acrylic polymer A, the MMA/(2EHA+BA) weight ratio, that is a ratio of the combined weight of two polyMMA blocks to the weight of poly(2EHA/BA) block, was 18/82. The acrylic polymer A had a Mw of $10 \times 10^4$ and a Mn of $8.4 \times 10^4$ at a Mw/Mn of 1.21.

(Acrylic Polymer B)

As the acrylic polymer B, was used an acrylic block copolymer having a triblock structure of MMA-2EHA/BA-MMA. The 2EHA to BA weight ratio in the poly(2EHA/BA) block was 50/50. In the acrylic polymer A, the MMA/(2EHA+BA) weight ratio, that is a ratio of the combined weight of two polyMMA blocks to the weight of poly(2EHA/BA) block, was 19/81. The acrylic polymer B had a Mw of $5 \times 10^4$ and a Mn of $4.4 \times 10^4$ at a Mw/Mn of 1.13.

Experiment 1

<Example 1>

In toluene, were mixed 100 parts of acrylic polymer, 15 parts of tackifier and 50 parts of plasticizer to prepare a solvent-based acrylic PSA composition with about 50 % NV (non-volatiles). As the acrylic polymer, were used the acrylic polymers A and B at a weight ratio of 80/20. As the tackifier, was used UH-115 (hydrogenated terpenophenolic resin) available from Yasuhara Chemical Co., Ltd. As the plasticizer, was used trade name MONOCIZER W-242 (diisononyl adipate) available from DIC Corporation. The PSA composition was applied to a face of a 38 μm thick PET support substrate sheet (approximately 8 cm wide) and allowed to dry at 100 °C for two minutes to fabricate a PSA sheet having an approximately 50 μm thick PSA layer formed on one face of the support substrate. The resulting PSA sheet was wound around the surface of a drum-shaped synthetic resin holding member (20 mm diameter) to form a PSA sheet roll. The holding member was attached in a freely rotatable (rolling) state to an end of a grip member to make a cleaner having a structure as schematically illustrated in Figs. 1 and 2.

Each of the PSAs in Example 1 here and Examples 2 to 10 below contains acrylic polymer A/acrylic polymer B/tackifier at a weight ratio of 80/20/15,

<Example 2>

To 100 parts of the acrylic polymer (the combined amount of acrylic polymer A and acrylic polymer B), was added 1.7 parts of the antimicrobial agent. Otherwise in the same manner as Example 1, a PSA sheet was fabricated, having a PSA layer with an antimicrobial agent concentration of 1.0 % formed on one face of the support substrate. As the antimicrobial agent, was used didecyldimethylammonium trifluoromethanesulfonate ($[(CH_3)_2N(C_{10}H_{21})_2]^+ CF_3SO_3^-$ under trade name NEOGERMI DFS available from Sanyo Chemical Industries, Ltd.). Using this PSA sheet, but otherwise in the same manner as Example 1, a cleaner according to Example 2 was fabricated. As shown in Table 1, the PSA forming the PSA layer of the cleaner according to Example 2 contained 1.7 parts of antimicrobial agent to 50 parts of plasticizer; and therefore, the antimicrobial agent content Wa to plasticizer content Wp ratio (Wa/Wp) is determined to be 0.034. As for the other examples, the Wa/Wp values can also be determined in the same manner.

<Examples 3 and 4>

The amounts of the antimicrobial agent used to 100 parts of the acrylic polymer were changed so that their concentrations in PSA were 5.0 % and 10.0 %, respectively. Otherwise in the same manner as Example 2, cleaners according to Examples 3 and 4 were fabricated.

<Examples 5 and 6>

No plasticizer was used. The amounts of the antimicrobial agent used to 100 parts of the acrylic polymer were adjusted so that the concentrations in PSA were 5.0 % and 10.0 %, respectively. Otherwise in the same manner as Examples 3 and 4, cleaners according to Examples 5 and 6 were fabricated.

The cleaners obtained in Examples 1 to 6 were subjected to the following test.

[Ability to provide antimicrobial properties]

From the PSA sheet according to each Example, a 50 mm by 50 mm square piece was cut out and adhered to a sterile culture dish. At one hour after adhered, the PSA sheet was peeled off from the sterile culture dish. With the area of the sterile culture dish where the PSA sheet had been adhered being the test surface, the sterile culture dish was subjected to a test for antimicrobial properties based on JIS Z 2801 "Antimicrobial products-Test for antimicrobial activity and efficacy" to determine the antimicrobial activity value (R) by the equation shown below.

[Math 1]

$$R=(U_t - U_0) - (A_t - U_0)=U_t - A_t$$

$R$ : Antimicrobial activity value
$U_o$ : Average of logarithmic values of viable cell counts of non-processed test piece immediately after inoculation
$U_t$ : Average of logarithmic values of viable cell counts of non-processed test piece at 24 hours
$A_t$ : Average of logarithmic values of viable cell counts of antimicrobial treated test piece at 24 hours

Here, the "non-processed test piece" corresponds to the surface of the sterile culture dish to and from which the PSA sheet according to Example 1 was adhered and removed after one hour; the "antimicrobial treated test piece" corresponds to the surface of the sterile culture dish to and from which the PSA sheet according to one of Examples 2 to 6 was adhered and removed after one hour. The initial microbial concentrations were $3.2 \times 10^4$ (CFU/mL) for Staphylococcus aureus and $6.1 \times 10^5$ (CFU/mL) for E. coli.

Table 1 shows the results as follows: E (excellent antimicrobial properties) when $R \geq 2.0$, S (significant antimicrobial properties) when $1.0 \leq R < 2.0$, and P (poor or no antimicrobial properties) when $R < 1.0$.

[Fingerprint Removability Test]

A smartphone (docomo NEXT series XPERIA® Z SO-02E available from Sony Mobile Communications AB) was obtained, with protection film applied to its display (a smooth surface made of aluminosilicate glass). As the protection film, protection film included with a jacket (a hard coating, gradation, shell jacket available from Ray-Out Co., Ltd.) for the smartphone was used. The protection film surface was thoroughly wiped with a non-woven fabric wipe (waste cloth) to remove any dirt.

Subsequently, sebum components on the tester's face (cheeks) were rubbed onto a finger (index finger) and the finger was firmly pressed on the protection film surface for two seconds to transfer the organic dirt (fingerprint) formed of sebum components on the finger to the protection film.

The protection film surface with the fingerprint was then cleaned with the cleaner according to each example. In particular, the PSA sheet roll of the cleaner according to each example was allowed to rotate five times in one direction over the protection film surface. The rotational speed was about 0.5 m/sec and the pressure applied by the operator for the rotation was about 700 g. After every rotation of the PSA sheet roll, the surface of the cleaned protection film was visually inspected and the fingerprint removability at the particular point was graded according to the following five levels.

5 points: The fingerprint completely disappeared.
4 points: The fingerprint mark mostly disappeared.
3 points: The fingerprint mark partially disappeared.
2 points: The fingerprint mark faded, but did not disappear.
1 point: No changes were observed in the fingerprint intensity.

The test was conducted with sebum of three testers A (female, 24 years old), B (male, 26 years old) and C (male, 24 years old). With respect to each tester, the PSA sheet roll was rotated five times to determine a total score of fingerprint removability. For instance, with respect to the tester A, if the resulting fingerprint removability test scores are 1 point, 1 point, 2 points, 3 points and 4 points for the first through the fifth round, the total score is 11 points. The total scores with respect to the testers A, B and C were further combined to determine the test score of fingerprint removability of the cleaner according to each Example. The test score of the cleaner according to Example 1 and the test scores of the cleaners according to the other Examples were compared (The greater the test score is, the better the fingerprint removability can be). In Table 1, the results are shown as follows: G (good fingerprint removability) when the difference from the test score of Example 1 was 1 or less; E (excellent fingerprint removability) when the test score was higher by at least 2 than Example 1; P (poor fingerprint removability) when the test score was lower by at least 2 than Example 1.

[Adhesive Strength]

Based on the 180° peel test described above, the PSA sheet according to each example was measured for 180° peel strength. In particular, the PSA sheet according to each example was cut into a 200 mm by 25 mm strip to obtain a test piece. The test piece was adhered over its sticky surface (the PSA layer surface) to a stainless steel (SUS304) plate with a 2 kg roller moved back and forth once. This was stored in an environment at 23 °C and 50 % RH for 30 minutes. Based on JIS Z0237, using a tensile tester, in the environment at 23 °C and 50 % RH, the 180° peel strength (N/25mm) to SUS was determined at a peel angle of 180° at a tensile speed of 1000 mm/min. Measurements were made with TENSILON available from Shimadzu Corporation. The results are shown in Table 1.
[Table 1]

Table 1

| | | Ex. 1 | Ex. 2 | Ex. 3 | Ex. 4 | Ex. 5 | Ex. 6 |
|---|---|---|---|---|---|---|---|
| PSA composition | Plasticizer (parts) | 50 | 50 | 50 | 50 | - | - |
| | Antimicrobial agent (parts) | - | 1.7 | 8.8 | 18.4 | 6.1 | 12.9 |
| | Concentration of antimicrobial agent | - | 1.0 % | 5.0 % | 10.0 % | 5.0 % | 10.0 % |
| | Wa/Wp | - | 0.034 | 0.174 | 0.368 | - | - |
| Ability to provide antimicrobial properties | Staphylococcus aureus | P | P | E | E | E | E |
| | E. coli | P | P | P | E | E | E |
| Fingerprint removability | | G | E | E | G | P | P |
| Adhesive strength to SUS (N/25mm) | | 0.17 | 0.10 | 0.01 | 0.05 | 0.51 | 0.09 |

As shown in Table 1, the cleaners according to Examples 3 and 4 both exhibited fingerprint removability (i.e. organic dirt removability) comparable to or greater than that of Example 1 as well as good abilities to provide antimicrobial properties against at least Staphylococcus aureus. The cleaner according to Example 4 showed good abilities to provide antimicrobial properties against both Staphylococcus aureus and E. coli. The cleaners of Examples 3 and 4 were both easy to release and provided good dirt-collecting workability.

In comparison between Examples 3 and 5, it can be seen that at the same concentration of antimicrobial agent, the ability of PSA to provide antimicrobial properties increased when the composition was free of plasticizer. With respect to the ability to provide antimicrobial properties against E. coli, the antimicrobial activity value (R) of Example 6 was higher than the R value of Example 4. In other words, in comparison between Examples 4 and 6, the ability to provide antimicrobial properties also increased when the composition was free of plasticizer. With respect to the compositions of Examples 2 and 4, in absence of plasticizer, the fingerprint removability decreased (Examples 5 and 6). The PSA according to Example 5 showed somewhat high adhesive strength as well. It is noted that with respect to a PSA sheet similarly fabricated using a PSA composition that comprised the same antimicrobial agent at the same concentration, but was free of plasticizer, the adhesive strength value was as high as 7.9 N/25mm.

Experiment 2

<Examples 7 and 8>

The amount of the plasticizer used per 100 parts of the acrylic polymer was changed to 30 parts. The amounts of the antimicrobial agent used to 100 parts of the acrylic polymer were changed so that their concentrations in PSA were 5.0 % and 7.5 %, respectively. Otherwise in the same manner as Example 2, cleaners according to Examples 7 and 8 were fabricated.

<Examples 9 and 10>

The amounts of the antimicrobial agent used to 100 parts of the acrylic polymer were changed so that their concentrations in PSA were 7.5 % and 15.0 %, respectively. Otherwise in the same manner as Example 2, cleaners according to Examples 9 and 10 were fabricated.

The cleaners obtained in Example 7 to 10 were tested for the ability to provide antimicrobial properties, fingerprint removability and adhesive strength in the same manner as in Experiment 1 except for the followings. In testing the ability to provide antimicrobial properties, the surfaces of sterile culture dishes upon application of the PSA sheets followed by immediate removal thereof were used as the test surfaces. As for the non-processed test piece in determining the antimicrobial activity values (R), was used the surface of a sterile culture dish upon application of the PSA sheet according to Example 1 followed by immediate removal thereof. The initial microbial concentrations were $1.1 \times 10^5$ (CFU/mL) for Staphylococcus aureus and $1.4 \times 10^5$ (CFU/mL) for E. coli. The results are shown in Table 2.

[Table 2]

Table 2

| | | Ex. 7 | Ex. 8 | Ex. 9 | Ex. 10 |
|---|---|---|---|---|---|
| PSA composition | Plasticizer (parts) | 30 | 30 | 50 | 50 |
| | Antimicrobial agent (parts) | 7.7 | 11.8 | 13.5 | 29.3 |
| | Concentration of antimicrobial agent | 5.0 % | 7.5 % | 7.5 % | 15.0 % |
| | Wa/Wp | 0.257 | 0.393 | 0.270 | 0.586 |
| Ability to provide antimicrobial properties | Staphylococcus aureus | E | E | S | E |
| | E. coli | E | E | S | E |
| Fingerprint removability | | G | E | E | E |
| Adhesive strength to SUS (N/25mm) | | 0.14 | 0.17 | 0.06 | 0.07 |

As shown in Table 2, the cleaners of Examples 7 to 10 all exhibited fingerprint removability (i.e. organic dirt removability) comparable to or greater than that of Example 1 as well as significant abilities to provide antimicrobial properties against both Staphylococcus aureus and E. coli. The cleaners of Examples 7 to 10 were all easy to release and provided good dirt-collecting workability. The cleaners of Examples 7, 8 and 10 showed greater abilities to provide antimicrobial properties. In comparison between Examples 8 and 9, it can be seen that at the same concentration of antimicrobial agent (7.5 %), with decreasing amount of plasticizer, the ability of PSA to provide antimicrobial properties increased.

To evaluate the antimicrobial properties of the PSA itself according to Examples 1 to 10, from the PSA sheets according to the respective Examples, 50 mm by 50 mm square pieces were cut out as test pieces (with the PSA layer surfaces used as the test surfaces) and tested for antimicrobial properties in the same manner. With the PSA sheet of Example 1 being a non-processed test piece and the PSA sheets of Examples 2 to 10 being antimicrobial treated test pieces, the antimicrobial activity values (R) of the respective PSAs were determined. As a result, the PSAs according to Examples 2 to 10 all had R values of 3.0 or higher, exhibiting good antimicrobial properties. In other words, when providing antimicrobial properties to PSA itself (unlike when providing antimicrobial properties to a surface subject to cleaning), the presence of plasticizer and its amount if any did not give rise to a difference in antimicrobial properties. These results support that with respect to a plasticizer/antimicrobial agent-containing PSA that provides antimicrobial properties to a surface being cleaned upon contact with the PSA (i.e. a PSA having an ability to provide antimicrobial properties), studies need to be conducted based on an idea different from the case where antimicrobial properties are provided to PSA itself.

Although specific embodiments of the present invention have been described in detail above, these are merely for illustrations and do not limit the scope of claims. The art according to the claims includes various modifications and changes made to the specific embodiments illustrated above.

[Reference Signs List]

1 portable device (article)
2 surface (display)
10 sticky cleaner
20 holding member
30 PSA sheet roll
31 PSA sheet (dirt-collecting member)
32 PSA layer
36 support substrate
40 grip member
42 handle
50 organic dirt

## Claims

1. A sticky cleaner for antimicrobial treatment used for removal of organic dirt deposited on an article surface and for antimicrobial treatment of the surface, wherein
the sticky cleaner comprises a dirt-collecting member that collects organic dirt upon contact with the article surface,
the dirt-collecting member comprises a pressure-sensitive adhesive in an area that comes in contact with the article surface, and
the pressure-sensitive adhesive comprises a base polymer and an antimicrobial agent.

2. The sticky cleaner according to Claim 1, wherein the surface-contacting area of the dirt-collecting member exhibits an adhesive strength of 1 N/25mm or less.

3. The sticky cleaner according to Claim 1 or 2, wherein the pressure-sensitive adhesive comprises the antimicrobial agent at a ratio of 2 % to 50 % by weight.

4. The sticky cleaner according to any one of Claims 1 to 3, wherein the pressure-sensitive adhesive further comprises a plasticizer, with the plasticizer content being 5 parts to 100 parts by weight to 100 parts by weight of the base polymer.

5. The sticky cleaner according to Claim 4, wherein the pressure-sensitive adhesive has an antimicrobial agent content Wa to plasticizer content Wp ratio (Wa/Wp) of 0.10 to 1.0.

6. The sticky cleaner according to any one of Claims 1 to 5, wherein the pressure-sensitive adhesive comprises an acrylic polymer as the base polymer.

7. The sticky cleaner according to Claim 6, wherein the base polymer is an acrylic block copolymer having a hard segment (A) and a soft segment (B) in one molecule.

8. The sticky cleaner according to Claim 7, wherein the soft segment (B) is formed with monomer units including an alkyl acrylate whose alkyl group has 6 or more carbon atoms.

9. The sticky cleaner according to any one of Claims 1 to 8, wherein the sticky cleaner comprises a cylindrical rolling member, and the dirt-collecting member is arranged along the lateral surface of the rolling member.

10. The sticky cleaner according to any one of Claims 1 to 9, used for removing sebum dirt as the organic dirt.

11. The sticky cleaner according to any one of Claims 1 to 10, wherein the article is a portable device having a display with a surface formed of glass or synthetic resin.

FIG.1

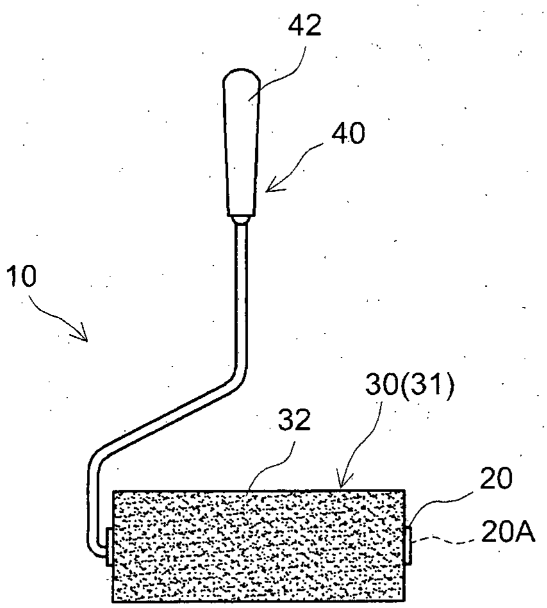


FIG.2

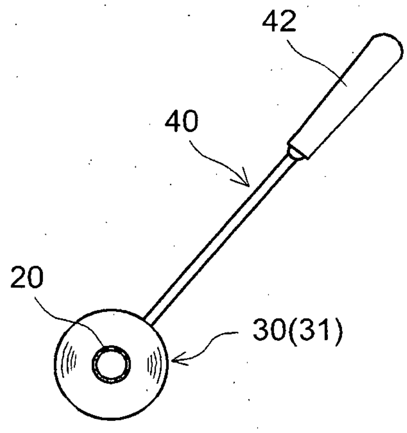

FIG.3

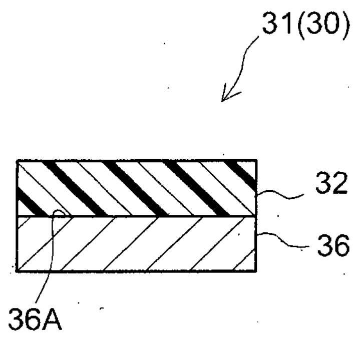

FIG.4

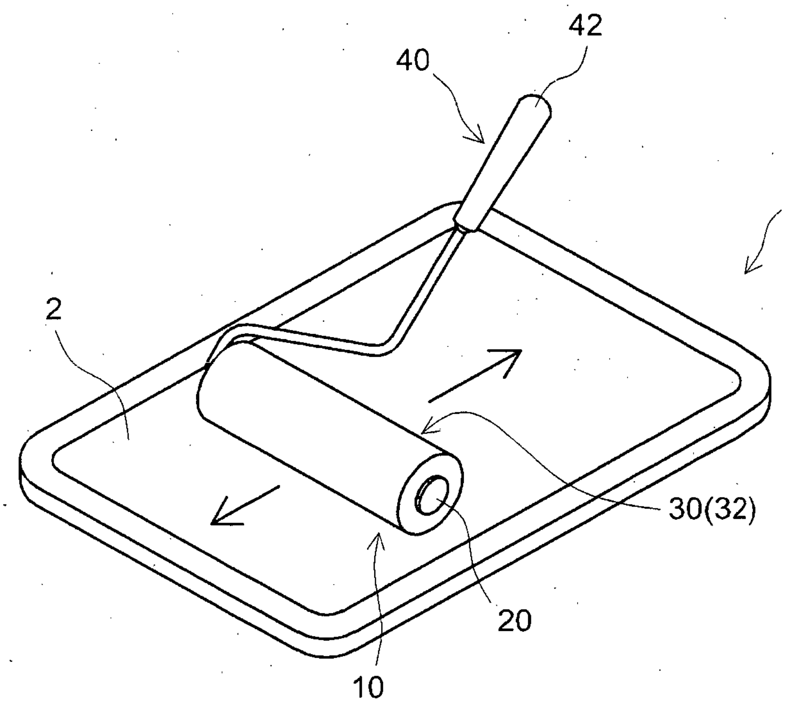

FIG.5

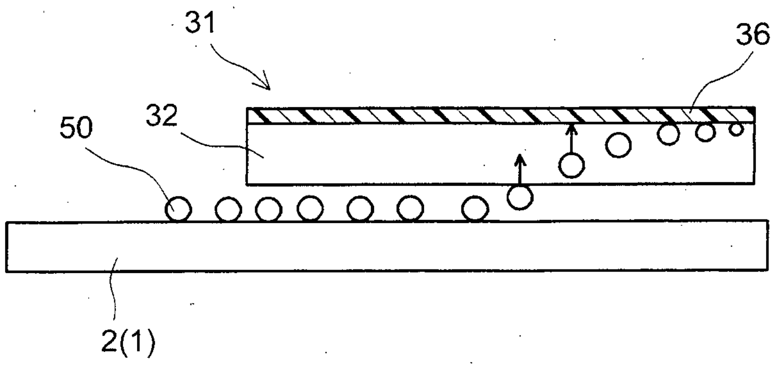

## INTERNATIONAL SEARCH REPORT

International application No.
PCT/JP2015/085616

A. CLASSIFICATION OF SUBJECT MATTER
*A47L25/00*(2006.01)i, *C09J11/06*(2006.01)i, *C09J133/00*(2006.01)i

According to International Patent Classification (IPC) or to both national classification and IPC

B. FIELDS SEARCHED

Minimum documentation searched (classification system followed by classification symbols)
A47L25/00, C09J11/06, C09J133/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2014-64833 A (Osada Corp.), 17 April 2014 (17.04.2014), paragraphs [0016] to [0021] (Family: none) | 1-11 |
| Y | WO 2013/015075 A1 (Nitoms, Inc.), 31 January 2013 (31.01.2013), paragraphs [0010], [0013], [0025] to [0039] & JP 2014-221422 A & US 2013/0125322 A1 & EP 2737840 A1 & CN 103108581 A & KR 10-2014-0045279 A & TW 201311200 A | 1-11 |
| Y | JP 8-24206 A (Nitto Denko Corp.), 30 January 1996 (30.01.1996), paragraphs [0012] to [0017], [0023] to [0027], [0033] (Family: none) | 3-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 March 2016 (04.03.16) | 22 March 2016 (22.03.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014259505 A
- WO 2013015075 A
- WO 2014115632 A
- JP 2001234146 A
- JP H11323072 B